(19) Europäisches Patentamt
European Patent Office
Office européen des brevets

(11) **EP 1 494 546 B1**

(12) **EUROPÄISCHE PATENTSCHRIFT**

(45) Veröffentlichungstag und Bekanntmachung des Hinweises auf die Patenterteilung:
**12.10.2005 Patentblatt 2005/41**

(51) Int Cl.⁷: **A23L 1/216**, A23L 2/74, A23L 1/304

(21) Anmeldenummer: **03718536.0**

(22) Anmeldetag: **17.04.2003**

(86) Internationale Anmeldenummer:
**PCT/AT2003/000114**

(87) Internationale Veröffentlichungsnummer:
**WO 2003/086102 (23.10.2003 Gazette 2003/43)**

(54) **VERFAHREN ZUR LEBENSMITTELTECHNISCHEN GEWINNUNG VON KARTOFFELSAFT-PRODUKTEN**

METHOD FOR THE PRODUCTION OF POTATO JUICE PRODUCTS BY MEANS OF FOOD TECHNOLOGY

PROCEDE D'OBTENTION PAR DES TECHNIQUES RELATIVES AUX PRODUITS ALIMENTAIRES DE PRODUITS A BASE DE JUS DE POMME DE TERRE

(84) Benannte Vertragsstaaten:
**AT BE BG CH CY CZ DE DK EE ES FI FR GB GR HU IE IT LI LU MC NL PT RO SE SI SK TR**

(30) Priorität: **18.04.2002 AT 6012002**

(43) Veröffentlichungstag der Anmeldung:
**12.01.2005 Patentblatt 2005/02**

(73) Patentinhaber: **vis-vitalis Lizenz- und Handels GmbH**
**5081 Anif (AT)**

(72) Erfinder:
• **KÖSSLER, Peter**
  **A-5571 Mariapfarr (AT)**
• **FUCHS, Norbert**
  **A-5571 Mariapfarr (AT)**

(74) Vertreter: **Sonn & Partner Patentanwälte**
**Riemergasse 14**
**1010 Wien (AT)**

(56) Entgegenhaltungen:
DE-A- 19 755 426        GB-A- 1 520 738

• **DATABASE WPI Section Ch, Week 197934 Derwent Publications Ltd., London, GB; Class D13, AN 1979-62112B XP002255668 & JP 54 086653 A (ASAHI CHEM IND CO LTD), 10. Juli 1979 (1979-07-10)**

**Beschreibung**

Die Erfindung betrifft ein Verfahren zur lebensmitteltechnischen Gewinnung von Kartoffelsaft-Produkten.

Der Säure-Basen-Haushalt des menschlichen Organismus ist als lebensnotwendiges System darauf ausgerichtet, den intrazellulär anfallenden Überschuss an Säuren permanent aus dem Stoffwechsel zu eliminieren. Während das in einer täglichen Menge von etwa 13.000 mmol anfallende $CO_2$ als flüchtige Säure über die Atmung ausgeschieden wird, fallen täglich zusätzlich 40 bis 60 mmol an Protonen aus nichtflüchtigen Säuren an. Diese Säuren (aber auch $CO_2$) werden in einem gesunden Organismus über verschiedene Puffer-Systeme reguliert, um den pH-Wert im Extrazellulärraum und im Blut weitgehend konstant zu halten. Respiratorische bzw. metabolische Abweichungen von physiologischen pH-Bereichen (Acidosen bzw. Alkalosen) stellen lebensbedrohliche Zustände dar und sind daher im Rahmen der Akutmedizin entsprechend zu therapieren.

Als weniger lebensbedrohlich, aber als "Milieu-Ursache" zahlreicher Erkrankungen wird dagegen die "latente Bindegewebs-Acidose" diskutiert, eine relative Übersäuerung des Interstitiums bzw. eine Überlastung der basischen Pufferkapazitäten des Bindegewebes durch endogen und/oder exogen anfallende Stoffwechsel-Säuren. Erkrankungen des Magen/Darm-Traktes, Leber- und Pankreas-Erkrankungen, Herz/Kreislauf-Erkrankungen, Asthma bronchiale, Diabetes mellitus, Migräne, Osteoporose, rheumatische Erkrankungen, Haarausfall, immunologische Erkrankungen wie Krebs, Niereninsuffizienz, Hauterkrankungen, neurologische Beschwerden, Schwangerschaftserbrechen, Durchblutungsstörungen sowie eingeschränkte Leistungsfähigkeit bei Sport und Schwerarbeit werden mit latenten Bindegewebs-Acidosen assoziiert.

Solanum tuberosum (Kartoffel, Erdapfel) wurde ursprünglich aus Südamerika nach Europa importiert und zählt hier mittlerweile zu den wichtigsten Grundnahrungsmitteln. Die Kartoffelknolle wird vor allem aufgrund ihres Gehaltes an leicht verdaulicher Stärke, aufgrund ihres hochwertigen Aminosäure-Musters und aufgrund des Gehaltes an natürlichen Vitaminen geschätzt. Weniger bekannt ist der Gehalt an Elektrolyten (Kalium-, Magnesium- und Calcium-Ionen), die vor allem in Form organischer Salze in der Kartoffelknolle vorliegen. Aufgrund ihres hohen Gehaltes an "basischen bzw. Basen-bildenden" Elektrolyten zählt die Kartoffel zu den Basen-bildenden Grundnahrungsmitteln. Basen-bildende Lebensmittel dienen dazu, einerseits den endogenen Ausstoß an nichtflüchtigen Säuren zu reduzieren, andererseits dazu, anfallende Stoffwechsel-Säuren zu neutralisieren und damit einer renalen Ausscheidung zuzuführen.

Im Zuge des Aufbereitungsverfahrens von Kartoffeln zu Kartoffel(press)saft sind jedoch mehrere störende Prozesse zu beobachten und zu kontrollieren: So sind Phenoloxidasen in der Lage, phenolische Verbindungen im Kartoffelsaft, wie Anthocyanidine, Flavone und Flavonole zu braun-grauen Polymeren zu oxidieren und daher zu einer störenden enzymatischen Verfärbung des Produktes zu führen.

Weiters werden auch die ernährungswissenschaftlich wichtigen Inhaltsstoffe der Kartoffel teilweise durch Lagerung erheblich in ihrer Aktivität reduziert: So hängt auch der Vitamin C-Gehalt von Kartoffeln sehr stark von deren Erntezeitpunkt und den Lagerbedingungen ab, beispielsweise hat eine Kartoffel, die nach sechsmonatiger Lagerung im März ohne Schale gekocht wird, nur mehr 2-3 mg Vitamin C auf 100 g, was nur mehr etwa einem Zehntel des ursprünglichen Frischewertes entspricht.

Aufgabe der vorliegenden Erfindung war daher, ein lebensmitteltechnisch anwendbares Verfahren zur Gewinnung von Kartoffelsaft-Produkten zur Verfügen zu stellen, bei welchem ein Produkt mit hohem Gehalt an basischen bzw. Basen-bildenden Elektrolyten erhalten wird, welches Produkt stabil ist, und neben einem hohen Elektrolytgehalt auch einen hohen Anteil an gelösten organischen Kohlenstoffverbindungen aufweist.

Die vorliegende Erfindung betrifft daher ein Verfahren zur lebensmitteltechnischen Gewinnung von Kartoffelsaft-Produkten, gekennzeichnet durch die folgenden Schritte:

- Bereitstellen von Kartoffelpresssaft
- Abfiltrieren von Faser- oder Stärkerückständen über Mikrofilter
- vorzugsweise Ultrafiltration des Filtrates
- Elektrodialyse des Mikro- bzw. Ultrafiltrates, und
- gegebenenfalls Trocknen des Elektrodialysates unter Zugabe von Silikat-hältigen Trägerstoffen.

Überraschenderweise konnte mit der Kombination an Verfahrensschritten im erfindungsgemäßen Verfahren der elektrolytische Anteil des Kartoffel-Frischsaftes in effizienter Weise von den Hauptbestandteilen der Kartoffelknolle, nämlich von Kohlehydraten und Stärke (etwa 86% der Trockensubstanz) sowie von Proteinen und freien Aminosäuren (etwa 12-13% der Trockensubstanz) getrennt werden. Dabei wird zunächst der Kartoffelpresssaft (oder Kartoffel-Frischsaft) zur Verfügung gestellt. Die Art und Weise, in der dieser Saft bereitgestellt wird, ist nicht kritisch; zahlreiche auch industriell taugliche Verfahren stehen dem Fachmann hierfür zur Verfügung. Auch für den Labormaßstab geeignete Verfahren sind bekannt. Beispielsweise können gewaschene und geschälte Kartoffel mechanisch zerkleinert und in einem handelsübliche Entsafter entsaftet werden. Bei der Saftherstellung entstehende Feststoffe, insbesondere Kartoffelstärke als Hauptmenge, werden in der Regel zunächst durch einfache Sedimentation (meist bei Kühlung, z. B. 4°C) abgetrennt. Der dabei entstehende Kartoffelpresssaft wird erfindungsgemäß zunächst über an sich bekannte Mikrofiltration von allfällig noch vorhandenen Feinrückständen, wie Faser- oder Stärkerückständen, gereinigt.

Im Anschluss daran eignet sich im erfindungsgemäßen Verfahren ganz besonders das Vorsehen einer Ultrafiltration, da damit Probleme durch Belegung der Membranen, also sogen. "Fouling-Phänomene" bei der nachfolgenden Elektrodialyse vermieden werden können. Mit Hilfe der Ultrafiltration können höher molekulare Bestandteile schon vor der Elektrodialyse abgetrennt werden.

Nach der erfindungsgemäß vorgesehenen Mikrofiltration und der bevorzugten Ultrafiltration folgt im vorliegenden Verfahren der Schritt der Elektrodialyse.

Im Gegensatz zur Mikro- oder Ultrafiltration, wo die gelösten Stoffe ausschließlich nach ihrer Teilchengröße, unabhängig von ihren chemischen und elektrochemischen Eigenschaften, aufgetrennt werden, erfolgt bei der Elektrodialyse eine ladungsabhängige Stoffauftrennung. Die Elektrodialyse ist ein Membranverfahren, bei welchem unter Einwirkung des elektrischen Feldes alle ionogenen Komponenten beeinflusst werden. Diese können somit spezifisch gemäß ihrer Ladung separiert werden. Die verschiedenen Ionen können dabei durch selektive Ionenaustauschmembranen in Kationen und Anionen separiert werden, wobei als treibende Kraft ein elektrisches Potentialgefälle wirkt. In Folge des angelegten elektrischen Potentials wandern die gelösten Ionen durch die Membranen auf die entsprechende Elektrode zu, so dass auf der einen Seite der Membrane eine verdünnte Lösung, das sogen. Diluat, zurückbleibt, und auf der anderen Seite die Ionen angereichert werden im sogen. Konzentrat.

Vorzugsweise wird dabei ein Membranstapel verwendet, wobei die einzelnen Membranen mittels Abstandhalter und die so entstandenen Räume mit Hilfe von Dichtrahmen voneinander und der Elektrodenkammer getrennt sind. Durch einen alternierenden Einsatz von Kationen- und Anionen-Austausch-Membranen resultiert eine Anreicherung der Ionen im sogen. Konzentrat-Kreislauf und eine entsprechende Abnahme an Ladungsträgern im sogen. Diluat-Kreislauf.

Die JP 53-115660 A betrifft ein Verfahren zur Behandlung eines Saftes, der Protein, anorganische Salze und ähnliche Inhaltsstoffe aufweist, wobei der Saft durch einen inversen Durchführungsseparator und ein Mikrofilter geleitet wird. Dabei wird eine Protein-angereicherte Saftfraktion gewonnen, welche an anorganischen Salzen abgereichert ist. Hierbei wird weder erwähnt, dass dieses Verfahren zur Herstellung von Kartoffelsaft oder Kartoffelsaft-Produkten verwendet werden kann, noch dass eine Elektrodialyse des erhaltenen Saftes vorgenommen wird. Weiters ist auch keine Ultrafiltration oder ein Trocknen des Elektrodialysats unter Zugabe von silikathältigen Trägerstoffen in diesem Dokument erwähnt.

Die Abreicherung anorganischer Salze gemäß der JP 53-115660 A stellt sogar das genaue Gegenteil der Wirkung dar, die mit dem erfindungsgemäßen Verfahren erreicht wird, bei welchem technologische Verfahrensschritte derart in Serie gesetzt werden, so dass die (basischen und Basen-bildenden) Elektrolyte (zu denen auch anorganische Salze gehören) aus Kartoffeln angereichert werden. Somit unterscheidet sich das dortige Verfahren nicht nur technologisch entscheidend vom erfindungsgemäßen Verfahren, sondern die mit dem Verfahren erzielte Wirkung ist das genaue Gegenteil der erfindungsgemäßen Wirkung.

Die JP 58-051880 A betrifft ein Verfahren zur Herstellung von Fruchtsäften, bei welchem der Saft über eine Ultrafiltrationsmembran geleitet wird. Dieses Verfahren wird aber nicht als für Kartoffelsaft anwendbar beschrieben. Weiters ist auch die Filtration über Mikrofilter und eine nachfolgende Elektrodialyse des Mikrofiltrats ebenso wenig erwähnt, wie das Trocknen des Elektrodialysats unter Zugabe von silikathältigen Trägerstoffen.

Das Ziel des Verfahrens gemäß der JP 58-051880 A ist, Säfte (insbesondere Fruchtsäfte) durch Ultrafiltration so spezifisch zu fraktionieren, dass damit die Sedimentation von Schwebstoffen minimiert wird und auf diese Weise gut lösliche Fruchtsaftkonzentrate gewonnen werden. Auch dieses Verfahren zielt daher nicht darauf ab, basische und Basen-bildende Elektrolyte aus Kartoffeln (die gerade nicht zu den Früchten zählen die unter die Definition des dort beschriebenen Verfahrens verwendet werden können) zu gewinnen.

DE 19755426 A beschreibt ein Verfahren zur Gewinnung von natürlich vorkommenden organischen Saüren aus filtriertem Rhabarberextrakt. Die Verwendung von kartoffelsaft wird nicht vorgeschlagen. Schließlich betrifft die JP 03-239701 A ein Verfahren zur Herstellung von Kartoffelstärke, bei welchem ein Kartoffelausgangsmaterial ("ground potato milk") deproteinisiert wird und zwar durch Anionenaustauscher mit tertiären Amin- oder quartären Amoniumgruppen. Im Gegensatz dazu ist das Verfahren gemäß der vorliegenden Erfindung nicht auf ein Verfahren zur Herstellung von Kartoffelstärke gerichtet, sondern auf ein Verfahren zur Herstellung von Kartoffelsaft-Produkten, bei welchen gerade der Proteinanteil, der gemäß diesem Dokument entfernt werden soll, beibehalten werden muss, um die ernährungsphysiologischen Zielsetzungen mit den erfindungsgemäßen Kartoffelsaft-Produkten realisieren zu können. Diese japanische Anmeldung führt somit eindeutig in eine völlig andere Richtung, da gemäß dem vorliegenden Verfahren neben den Proteinen vor allem die Stärkefraktion abgetrennt wird, um den Gehalt an basischen Elektrolyten und Spurenelementen optimal anzureichern, wohingegen das gemäß diesem Dokument geoffenbarte Verfahren lediglich zur Gewinnung flüssiger Kartoffelstärkekonzentrate als Lebensmittelgrundstoff zur Aromatisierung und Konsistenzgebung von Lebensmittel dient.

Das erfindungsgemäß erhaltene Elektrodialysat kann dann, wenn erwünscht, sofort als lebensmitteltechnischer Kartoffelsaft zu entsprechenden Lebensmittelprodukten fertiggestellt oder aber weiter aufgearbeitet oder - gegebenenfalls als Zwischenprodukt - gelagert werden. Für Lagerungszwecke empfiehlt sich allerdings eine Trocknung des Saftes. Um die Entstehung einer amorphen Masse bei diesem Trocknungsprozess zu vermeiden, werden bevorzugterweise bei der Trocknung Trägerstoffe zugesetzt, die selbstverständlich lebensmitteltechnisch tauglich sein sollten, d.h. in Lebensmitteln keine unerwünschten Eigenschaften herbeiführen. Besonders bewährt haben sich hierbei Silikat-hältige Trägerstoffe, insbesondere Siliziumdioxid enthaltende hochdisperse Silikate. Unter hochdispersen Silikaten versteht man Silikat-hältige Trägerstoffe mit großer Oberfläche (z.B. einer Oberfläche von über 50 $m^2$/g, von über 100 $m^2$/g oder von über 150 $m^2$/g), wie z.B. Aerosil 200 (Oberfläche 200 $m^2$/g) bzw. Aerosil 380 (Oberfläche 380 $m^2$/g).

Gemäß einer bevorzugten Ausführungsform werden dem Kartoffelpresssaft Stabilisierungsmittel zugesetzt. Günstig ist es, diese bereits entweder nach oder während der Pressung zuzugeben. Besonders bewährt haben sich hierbei die Verwendung von Zitronen bzw. Zitronen(press)saft oder Zitronensaft-Produkten, da diese als natürliche Antioxidantien in der Lage sind, unerwünschte Enzymaktivitäten im Kartoffelsaft, insbesondere verursacht durch Oxidasen, zu unterbinden. Sehr gute Ergebnisse werden im Rahmen des erfindungsgemäßen Verfahrens auch mit Ascorbinsäure, schwarzem Johannisbeersaft, Holunderblütenextrakt, Sanddornsaft, ganzen Zitronen oder ganzen roten Paprika erhalten. Von den insbesondere natürlichen Antioxidantien, die als Stabilisierungsmittel eingesetzt werden können, sind zwar wie erwähnt vor allem Zitronensaft oder Zitronensaft-Produkte besonders bevorzugt, jedoch ist die Optimierung des Stabilisierungsmittels im Einzelfall auch von der speziellen Kartoffelsorte bzw. der Art des Wachsens und der Lagerung abhängig.

Bevorzugterweise wird der Kartoffelpresssaft aus Kartoffeln bereitgestellt, die ein Verhältnis von Basen-bildenden zu Säurebildenden Komponenten von zumindest 1,5, bevorzugt davon über 3,5 aufweisen. Dieses Verhältnis kann von jedem Fachmann durch einfache Analytik anhand des Ausgangsmaterials festgestellt werden. Insbesondere sind erfindungsgemäß Kartoffelpresssäfte aus den Sorten Desiree und Ackersegen bevorzugt. Weitere bevorzugte Sorten sind Bintje, Desiree, Ostara und Planta. Jedoch sind auch andere festkochende (speckige), vorwiegend festkochende oder mehligkochende Kartoffel gut für das erfindungsgemäße Verfahren geeignet. In der Regel sollten aber die Ausgangsprodukte vor allem hinsichtlich ihrer (basischen) Elektrolytgehalte vorab untersucht und - auch chargenweise - ausgewählt werden, wobei dem oben erwähnten Verhältnis von Basen-bildenden zu Säure-bildenden Komponenten Rechnung getragen werden sollte. Dieses Verhältnis wird oft über das Calcium/Phosphor-Verhältnis charakterisiert, genauer ist hierbei aber die Ermittlung eines Quotienten der molaren Konzentrationen an Säure-bildenden Komponenten dividiert durch die Summe der molaren Konzentrationen an Basen-bildenden Komponenten. Zu den Säure-bildenden Komponenten zählen dabei Sulfat, Phosphat, Chlorid, Nitrat, Malat, Lactat, Tartrat, Citrat, Isocitrat und L-Ascorbat. Zu den Basen-bildenden Komponenten werden unter anderem Kalium, Magnesium, Calcium, Natrium, Ammonium, Eisen, Zink, Mangan, Kupfer, Selen, Nickel und Chrom gezählt.

Wie erwähnt, können durch Vorsehen eines Ultrafiltrationsschrittes allfällige Probleme ("fouling", Belegung von Membranen, ...) bei der Elektrodialyse vermieden werden. Vorzugsweise wird dabei ein Ultrafilter mit einer Ausschlussgrenze von unter 100.000 Da, bevorzugt unter 10.000 Da, insbesondere von etwa 1.000 Da, verwendet, wobei hierbei in der Regel mit Überdruck gearbeitet wird, bevorzugterweise mit 1,1 bis 10 bar, insbesondere mit etwa 2 bar.

Bei der Elektrodialyse hat sich erfindungsgemäß besonders die Verwendung von Niedrig-Diffusionsmembranen bewährt, wodurch sich Verschmutzung und Quellung der Elektrodialysemembran größtenteils (auch ohne Ultrafiltration) vermeiden lassen wie auch allfällige "Fouling-Prozesse".

Wie erwähnt, kann der erfindungsgemäß erhaltene Kartoffelpresssaft sofort lebensmitteltechnisch weiter verarbeitet werden. Eine Lagerung oder ein Transport in flüssigem Zustand ist jedoch bei größeren (industriellen) Mengen nicht immer erwünscht, so dass vorzugsweise ein Trocknungsprozess an das erfindungsgemäß Verfahren angehängt wird. Da bei herkömmlicher Trocknung aus Kartoffelsaft lediglich eine amorphe Masse wird, die industriell nur schwer handhabbar ist, wird erfindungsgemäß beim Trocknungsprozess bevorzugterweise hoch-disperses Siliziumdioxid zugegeben.

Bevorzugterweise werden dem erhaltenen und gegebenenfalls getrockneten Kartoffelsaft-Produkt ein oder mehrere weitere Mittel zugesetzt, vorzugsweise zumindest ein weiterer Gemüse- oder Fruchtsaft, ein oder mehrere Stabilisierungsmittel, insbesondere natürliche Antioxidantien, ein oder mehrere, insbesondere natürliche Geschmacks- oder Farbstoffe, ein oder mehrere Verdickungs, Rekonstituierungs- oder Elektrolytmittel oder Kombinationen dieser Mittel. Dem getrockneten Kartoffelsaft-Produkt können weiters auch Vitamine, Mineralstoffe, Spurenelemente oder sekundäre Pflanzenstoffe in beliebiger Qualität und Quantität, einzeln oder in Kombination zugesetzt werden. Rekonstituierungsmittel dienen dabei einer vollständigen und schnellen Wiederüberführung in den flüssigen Zustand; Elektrolytmittel verleihen dem zu rekonstituierenden Kartoffelsaft-Pulver zusätzliche Elektrolyte.

Vorzugsweise wird das Trocknen durch Sprüh- oder Walztrocknen vorgenommen. Ein Vorteil der Zugabe von Siliziumdioxid-Präparaten besteht auch darin, dass herkömmliche, industriell verwendbare Trocknungsanlagen verwendet werden können.

EP 1 494 546 B1

Gemäß einem weiteren Aspekt betrifft die vorliegende Erfindung auch ein Kartoffelsaft-Produkt, welches gemäß dem erfindungsgemäßen Verfahren erhältlich ist.

Je nach Konzentration des Saftes bzw. des Gehalts im Ausgangsmaterial enthält das erfindungsgemäße Kartoffelsaft-Produkt vorzugsweise 1000 mg/l oder mehr organische Bestandteile, vorzugsweise 2000 mg/l oder mehr, insbesondere 4000 mg/l oder mehr, vorzugsweise bestimmt als nicht ausblasbarer organischer Kohlenstoff.

Ein besonders bevorzugtes erfindungsgemäßes Kartoffelsaft-Produkt zeichnet sich dadurch aus, dass es ein Verhältnis von Basen-bildenden zu Säure-bildenden Komponenten von zumindest 2,5, bevorzugt von über 4, insbesondere von über 6 aufweist.

Weiters betrifft die vorliegende Erfindung auch die Verwendung von erfindungsgemäßen Kartoffelsaft-Produkten mit basischen bzw. Basen-bildenden Elektrolyten zur (Herstellung eines Mittels zur) Regulierung des Säure/Basen-Haushaltes im menschlichen oder tierischen Organismus. Das Mittel kann dann in einer geeigneten Dosis zur Balance des Säure/Basen-Haushaltes an Mensch oder Tier verabreicht werden.

Die Erfindung wird anhand der nachfolgenden Beispiele sowie der Zeichnungsfiguren, auf die sie selbstverständlich nicht eingeschränkt sein soll, näher erläutert. Es zeigen:

Fig.1 einen Überblick über ein bevorzugtes erfindungsgemäßes Verfahren,
Fig.2 das Funktionsprinzip der Elektrodialyse, wobei AEM die Anionen-Austausch-Membran bezeichnet und CEM die Kationen-Austausch-Membran, und

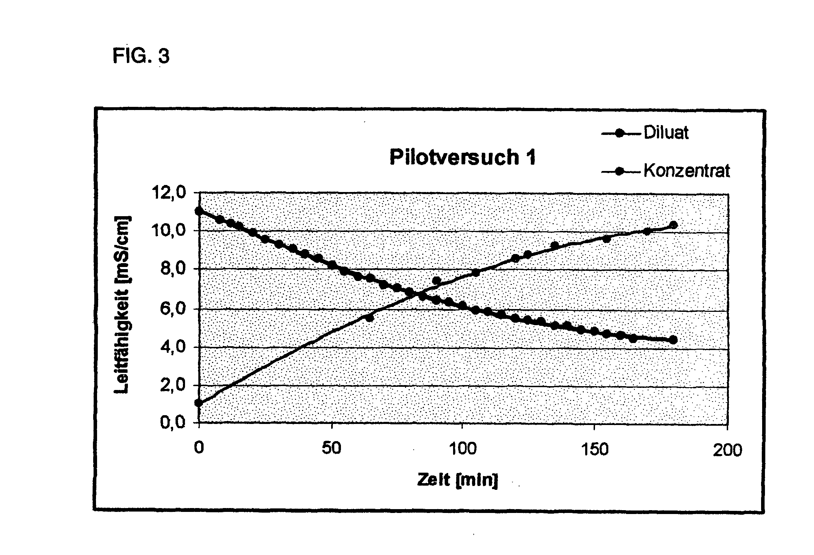

Fig.3 bis Fig.5 die Leitfähigkeit gegen die Zeit in Diluat und Konzentrat bei der Elektrodialyse in den Beispielen 1 bis 3.

## 1. STABILISIERUNGSVERSUCHE

### 1.1. Materialien

Ausgehend vom herkömmlichen Ansatz, zugelassene Antioxidantien zur Stabilisierung des Kartoffelpresssaftes zu verwenden, wurde untersucht, ob sich der Zusatz von ausgewähltem Pflanzenmaterial (Obst, Gemüse) zur Hemmung der Phenoloxidasenaktivität eignet. Die Auswahl dieser Zusätze erfolgt nach gesundheitsrelevanten, ökologischen, technologischen und ökonomischen Kriterien.

Soweit erhältlich wurde Obst/Gemüse aus kontrolliert biologischem Anbau verwendet, waren kbA-Qualitäten nicht verfügbar, wurde teilweise auf standardisierte, kommerziell erhältliche Pflanzensäfte (kbA) zurückgegriffen.

### Kartoffel-Probenmaterial

AGATA          österr. Heurige aus biologischer Landwirtschaft, Ernte 2001, festkochend
FRIESLANDER    österr. Heurige aus biol. Landwirtschaft, Ernte 2001, vorwiegend festkochend
BINTJE         aus österr. biol. Landwirtschaft, Ernte 2000, vorwiegend festkochend

### Substanzen für die Stabilisierungsversuche

• Octylgallat E311
• Ascorbinsäure E300
• $CO_2$ Begasung
• Broccoli Presssaft
• Holunderblütenextrakt (standardisiertes, kommerziell erhältliches Produkt)
• Kohl Presssaft
• Paprika grün Presssaft
• Paprika rot ganz
• Sanddornsaft (standardisiertes, kommerziell erhältliches Produkt)
• Schwarze Johannisbeere Saft (standard., kommerziell erhältliches Produkt)
• Zitrone ganz
• Zitronensaft

**Geräte**

Kartoffelschäler Edelstahl
Entsafterzentrifuge ELIN
Standard-Glaseprouvetten, Parafilm
Genormte Farbpalette
Canon Digitalkamera, Halogenlampe

**1.2 Methoden**

**Stabilisierungsversuche durch Zusätze <u>nach</u> dem Pressvorgang**

Aus den geschälten, gewaschenen Kartoffeln wurde mittels Entsafterzentrifuge unter raschem Durchsatz des Probenmaterials Kartoffelsaft gewonnen. Unmittelbar danach wurde der Saft in die Testgefäße übergeführt und mit dem jeweiligen Stabilisierungszusatz in unterschiedlichen Konzentrationen versetzt.

**Stabilisierungsversuche durch Zusätze <u>während</u> des Pressvorgangs**

Die geschälten, gewaschenen und grob gestückelten (halbiert bis geviertelten) Kartoffeln wurden möglichst homogen mit dem jeweiligen Zusatz (ca. 1 cm$^3$ große Gemüsestückchen) vermengt. Flüssige Zusätze wurden während der Zentrifugenentsaftung gleichmäßig zudosiert. Unter raschem Durchsatz wurden die Kartoffelsaft+Zusatz-Gemische gewonnen. Die Proben wurden sofort in Eprouvetten übergeführt.

**Versuchsablauf**

- Die Kartoffelsaft+Zusatz-Gemische werden in verschlossenen Glaseprouvetten gleicher Größe und gleicher Qualität vorgesehen.
- Die Farbwerte der Kartoffelsaft+Zusatz-Gemische sowie die der Referenzproben (ohne Zusatz) wurden mittels Farbtabellen-Vergleich bei Laborbeleuchtung sowie mittels Digitalkamera bei Halogenlampenlicht dokumentiert.
- Aufnahme der Farbwerte: Erstmals unmittelbar nach Probenbereitung, danach in Tagesintervallen
- Beobachtungszeitraum: 1 Woche

**Auswertung**

Die Farbwerte zu Versuchsbeginn wurden den nach einer Woche beobachteten Werten gegenübergestellt.

| +++ | keine oder nur sehr geringe Farbveränderung | - | nicht signifikante Verfärbungshemmung |
|---|---|---|---|
| ++ | mittlere Verfärbung | -- | starke Verfärbung |
| + | noch signifikante Verfärbungshemmung | --- | sehr starke Verfärbung (schwarzbraun) |

**1.3 Ergebnisse der Stabilisierungsversuche**

Die Ergebnisse der Stabilisierungsversuche sind in der nachfolgenden Tabelle 1 dargestellt.

## Ergebnisse der Stabilisierungsversuche

### Tabelle 1

| Stabilisierungsversuche durch Zusätze | | | | | |
|---|---|---|---|---|---|
| **NACH dem Pressvorgang** | **Zusätze %(v/v)** | **Anmerkung** | **Werte nach 1 Woche** | | **Interpretation** |
| Ascorbinsäure E300 | 0,01% bis 2, 5% | | +++ | => | zeigt gute Stabilisierung |
| Zitrone Presssaft | 0,01% bis 10% | wird heller | ++/+++ | => | zeigt gute Stabilisierung |
| Schwarzer Johannisbeersaft | 0, 05% bis 10% | Eigenfärbung | + | => | Stabilisierungspotential vorhanden |
| Holunderblütenextrakt | 0,05% bis 10% | | + | => | Stabilisierungspotential vorhanden |
| Sanddornsaft | 0,05% bis 10% | Ausflockung, Eigenfärbung | + | => | Stabilisierungspotential vorhanden |
| Octylgallate E311 | 0,01 % bis 2,5% | | - | => | geringes Stabilisierungspotential |
| Presssaft grüner Paprika | 0,05% bis 10% | Eigenfärbung | - | => | Versuche mit rotem Paprika |
| Presssaft aus Broccoli | 0, 05% bis 10% | Eigenfärbung | - | => | wurde nicht weiterverfolgt |
| Presssaft aus Kohl | 0, 05% bis 10% | Eigenfärbung | - | => | wurde nicht weiterverfolgt |
| **WÄHREND der Pressung** | **Zusätze %(w/w)** | | | | |
| Zitrone ganz (KbA, unbehandelt!) | 5% bis 10% | wird heller | +++ | => | zeigt sehr gutes Stabilisierungspotential; Optimierung hinsichtlich der Mindest-Einsatzkonzentration |
| Holunderblütenextrakt | 5% bis 10% | | +++ | => | zeigt sehr gutes Stabilisierungspotential; Optimierung hinsichtlich der Mindest-Einsatzkonzentration |

EP 1 494 546 B1

| Stabilisierungsversuche durch Zusätze | | | | | |
|---|---|---|---|---|---|
| Schwarzer Johannisbeersaft | 5% bis 10% | Eigenfärbung | ++/+++ | => | zeigt gutes Stabilisierungspotential; "heimischer" Rohstoff; Eigenfärbung ist zu berücksichtigen |
| Sanddornsaft | 5% bis 10% | starke Ausflockung, Eigenfärbung | ++/+++ | => | zeigt gutes Stabilisierungspotential; leichte Eigenfärbung sowie starke Ausflockung sind zu berücksichtigen |
| Roter Paprika ganz | 5% bis 10% | Eigenfärbung | ++/+++ | => | zeigt gutes Stabilisierungspotential; starke Eigenfärbung ist zu berücksichtigen; Nitrateintrag !? |
| Kartoffelsaft Referenzproben "gelagert" | - | | -- | => | starke Verfärbung innerhalb kürzester Zeit - schon während des Pressvorganges |
| Kartoffelsaft Referenzproben "erntefrisch" | - | | +/- | => | Presssaft aus erntefrischen Kartoffeln zeigt relativ gute Stabilität bei Kühlung und Luftabschluss |

EP 1 494 546 B1

## 2. SORTENAUSWAHL

Da davon auszugehen ist, dass unterschiedliche Kartoffelspecies (mehlig, speckig etc.) und regional-spezifische Besonderheiten das Verteilungsmuster der Inhaltsstoffe erheblich beeinflussen, wurden verschiedene in Österreich kultivierte Sorten im Hinblick auf die gewinnbaren Elektrolyte Kalium, Magnesium, Calcium, Natrium, Eisen, Zink, Mangan, Kupfer, Selen, Nickel, Chrom, Ammonium, die anorganischen Anionen Sulfat, Phosphat, Chlorid und Nitrat, sowie die organischen Anionen Ascorbat, Malat, Lactat, Tartrat, Citrat und Isocitrat untersucht.

2.1. Kartoffel-Probenmaterial

Tabelle 2

| Code | Solanum tuberosum | Herkunft | Ernte | Typ |
|------|-------------------|----------|-------|-----|
| L1 | Ostara | Lungau | 2000 | vorwiegend festkochend |
| L2 | Desiree | Lungau | 2000 | vorwiegend festkochend |
| S3 | Planta | Stainz | 2000 | vorwiegend festkochend |
| S4 | Desiree | Stainz | 2000 | vorwiegend festkochend |
| B5 | Bintje | Gratkorn | 2000 | vorwiegend festkochend |
| 01 | Ostara | Lungau | 2001 | vorwiegend festkochend |
| D1 | Desiree | Lungau | 2001 | vorwiegend festkochend |
| A1 | Ackersegen | Lungau | 2001 | mehlig |

## 2.2 Qualität des Probenmaterials

Sämtliches verwendetes Probenmaterial entsprach mindestens den für Speisekartoffeln vorgeschriebenen Kriterien nach BGB1. Nr. 76/1994, zuletzt geändert durch BGB1. Nr. 240/1997, Verordnung des Bundesministers für Land- und Forstwirtschaft über Qualitätsklassen für Speisekartoffeln, d.h äußerlich frisch und grob-optisch gesund, vorwiegend festkochend bzw. mehlig. Gemäß § 3 dieser Verordnung waren die hier eingesetzten Kartoffeln:

1. ganz,
2. gesund, insbesondere frei von Nass-, Braun- und Trockenfäule, von einem 25 % der Knollenoberfläche übersteigenden Oberflächenschorf, von einem 10% der Knollenoberfläche übersteigenden Tiefenschorf, von Hitze- oder Frostschäden, Eisenfleckigkeit, Hohl- oder Schwarzherzigkeit, starker Pfropfenbildung, starker Glasigkeit, starker Stippigkeit und starker Schwarzfleckigkeit,
3. sauber, das heißt nahezu frei von Erde oder Sand,
4. fest, das heißt nicht welk oder runzelig,
5. frei von anomaler äußerer Feuchtigkeit,
6. frei von fremdem Geruch oder Geschmack,
7. frei von starken Beschädigungen, Fraßstellen oder schweren Quetschungen,
8. frei von deutlich ergrünten Knollen und
9. frei von missgestalteten Knollen (Zwiewuchs, Kindelbildung usw.).

Weiters waren die Packstücke frei von fremden Bestandteilen, wie Erde, Sand oder losen Keimen, und grundsätzlich schalenfest.
Weiters wiesen die verwendeten Kartoffeln die folgenden Beschaffenheitsmerkmale auf (Klasse I):
Die Kartoffeln waren sortentypisch. Zulässig waren jedoch folgende Fehler:

a) leichte Grünfärbung auf höchstens 1/8 der Knollenoberfläche;
b) leichte oberflächliche Beschädigungen, die durch das normale Schälen entfernt werden können,
c) Beschädigungen oder Schwarzfleckigkeit, die nicht tiefer als 5 mm reichen und zu deren Beseitigung nicht mehr als 10% des Knollengewichtes erforderlich sind und

d) Keime mit einer Länge von höchstens 3 mm.

Die Einteilung der Speisekartoffeln nach dem Kochtyp wurde wie folgt vorgenommen:

1. Festkochende (speckige) Kartoffeln:

Agata*), Ditta, Evita, Exquisa, Julia, Linzer Delikatess, Naglemer Kipfler, Nicola, Novita, Punika, Sieglinde, Sigma, Sonja

2. Vorwiegend festkochende Kartoffeln:

Accent, Adora*), Berber*), Bettina, **Bintje,** Bionta,Celeste*), Christa*), Cinja, **Desiree,** Erstling*'), Frieslander*), Gina*), Goldsegen, Impala*), Isola, Jaerla*), Jetta, Linzer Gelbe, Minerva*), **Ostara*), Planta,** Platina, Quarta, Romina, Rosella, Rubinia*), Salenta, Sirtema*), Timate, Ukama*).

3. Mehligkochende (mehlige) Kartoffeln:

**Ackersegen,** Agria, Ares, Asterix, Aula, Cosima, Donald, Erntestolz, Fambo, Hermes, Mondial, Remarka, Russet Burbank, Satuma, Signal, Solara, Treff, VanGogh, Welsa.

**2.3 Methoden**

Zur Sortenauswahl wurde eine umfassende Analytik der für das erfindungsgemäße Produkt wertbestimmenden Inhaltstoffe durchgeführt.

Die gewaschenen und geschälten Kartoffeln wurden mechanisch zerkleinert und in einem handelsüblichen Entsafter entsaftet. Die Hauptmenge der Kartoffelstärke wurde durch eine mehrstündige Sedimentation bei +4°C abgetrennt. Der so erhaltene Stärke-arme Kartoffelpresssaft wurde wie folgt untersucht:

- Kationen (Kalium, Magnesium, Calcium, Natrium, Eisen, Zink, Mangan, Kupfer, Selen, Nickel und Chrom) wurden nach einem Säureaufschluss mit Schwefelsäure mittels ICP-MS (Massenspektrometrie mit induktiv gekoppeltem Plasma) quantifiziert
- Ammonium wurde nach einer Filtration photometrisch mittels Nessler's Reagenz detektiert
- Anorganischen Anionen (Sulfat, Phosphat, Chlorid und Nitrat) wurden nach einer Filtration mittels IC (Ionenchromatographie) und Leitfähigkeitsdetektion nachgewiesen
- Organischen Anionen (Malat, Lactat, Tartrat, Citrat und Isocitrat) wurden ebenfalls nach einer Filtration mittels IC (Ionenchromatographie) und Leitfähigkeitsdetektion nachgewiesen
- Ascorbat wurde photometrisch vor und nach einer enzymatischen Oxidation mit Ascorbat-Oxidase bestimmt
- Als Summenparameter für organische Bestandteile wurde der DOC (gelöster organischer Kohlenstoff) als NPOC (nicht ausblasbarer organischer Kohlenstoff) bestimmt.

Ausgehend von der in der Literatur häufig zu findenden Methode, die Säure- bzw. Basen-bildenden Eigenschaften von Lebensmitteln über das Calcium/Phosphor-Verhältnis zu charakterisieren, wurde diese Berechnungsvariante auf die Summenwerte aus der Analytik erweitert:

Zur Auswertung wurden die molaren Konzentrationen der Säure-bildenden Anionen auf jene der Basen-bildenden Kationen bezogen. Der so erhaltene Quotient wurde als Entscheidungskriterium für die Sortenauswahl herangezogen.

$$Q = \frac{\Sigma \text{ der molaren Konzentrationen an Säure-bildenden Komponenten}}{\Sigma \text{ der molaren Konzentrationen an Basen-bildenden Komponenten}}$$

Werten < 1 wird die physiologische Eigenschaft "Säure-bildend" und Werten > 1 "Basen-bildend" zugeordnet. Je höher der Wert, desto stärker Basen-bildend ist die Probe.

**2.4 Ergebnisse der Untersuchungen ausgewählter Kartoffelsorten**

Die ersten Untersuchungen wurden von den Kartoffelsorten Ostara und Desiree mit Herkunft Lungau, Planta und Desiree aus Stainz und Bintje aus Gratkorn aus der Ernte 2000 im Frühjahr 2001, also nach einer Lagerzeit von

*) Sehr früh reifende Kartoffeln

etwa 6 Monaten durchgeführt. Die Ergebnisse dieser Untersuchungen sind auf der folgenden Seite tabellarisch dargestellt (Tabelle 2a, b, c).

Ferner wurden die Sorten Ostara, Desiree und Ackersegen, geerntet im Lungau, auch unmittelbar nach der Ernte 2001 untersucht.

| | mg/mmol | L1 Ostara Ernte 2000 mg/l | L1 Ostara Ernte 2000 mmol/l | L2 Desiree Ernte 2000 mg/l | L2 Desiree Ernte 2000 mmol/l | S3 Planta Ernte 2000 mg/l | S3 Planta Ernte 2000 mmol/l | S4 Desiree Ernte 2000 mg/l | S4 Desiree Ernte 2000 mmol/l | B5 Bintje Ernte 2000 mg/l | B5 Bintje Ernte 2000 mmol/l |
|---|---|---|---|---|---|---|---|---|---|---|---|
| Sulfat | 96,06 | 1190 | 12,39 | 702 | 7,31 | 770 | 8,02 | 663 | 6,90 | 942 | 9,81 |
| Phosphat | 94,97 | 791 | 8,33 | 796 | 8,38 | 689 | 7,25 | 592 | 6,23 | 811 | 8,54 |
| Chlorid | 35,45 | 244 | 6,88 | 94 | 2,65 | 151 | 4,26 | 178 | 5,02 | 500 | 14,10 |
| Nitrat | 62,01 | 100 | 1,61 | 43 | 0,69 | 125 | 2,02 | 56 | 0,90 | 66,5 | 1,07 |
| Malat | 132,10 | | | | | | | | | | |
| Lactat | 89,08 | | | | | | | | | | |
| Tartrat | 148,10 | | | | | | | | | | |
| Citrat | 189,10 | | | | | | | | | | |
| Isocitrat | 189,10 | | | | | | | | | | |
| L-Ascorbat | 175,05 | | | | | | | | | | |
| **Summe säurebildend** | | | 29,21 | | 19,03 | | 21,55 | | 19,06 | | 33,52 |
| Kalium | 39,10 | 2880 | 73,66 | 2640 | 67,52 | 3200 | 81,84 | 3190 | 81,59 | 4667 | 119,36 |
| Magnesium | 24,31 | 257 | 10,57 | 252 | 10,37 | 230 | 9,46 | 257 | 10,57 | 358 | 14,73 |
| Calcium | 40,08 | 27,6 | 0,69 | 23,5 | 0,59 | 32,8 | 0,82 | 19,3 | 0,48 | 80,4 | 2,01 |
| Natrium | 22,99 | 11,8 | 0,51 | 13,4 | 0,58 | 13,1 | 0,57 | 8,6 | 0,37 | 25,9 | 1,13 |
| Ammonium | 18,04 | <0,05 | n.a. | <0,05 | n.a. | <0,05 | n.a. | <0,05 | n.a. | <0,05 | n.a. |
| Eisen | 55,84 | 2,07 | 0,04 | 3,95 | 0,07 | 4,33 | 0,08 | 3,44 | 0,06 | 2,9 | 0,05 |
| Zink | 65,30 | 2,82 | 0,04 | 2,88 | 0,04 | 3,6 | 0,06 | 2,84 | 0,04 | 5,01 | 0,08 |
| Mangan | 54,94 | 1,35 | 0,02 | 1,82 | 0,03 | 1,04 | 0,02 | 1,08 | 0,02 | 1,6 | 0,03 |
| Kupfer | 63,54 | 0,85 | 0,01 | 1,1 | 0,02 | 1,2 | 0,02 | 1,9 | 0,03 | 1,79 | 0,03 |
| Selen | 78,90 | <0,002 | n.a. | <0,002 | n.a. | <0,002 | n.a. | <0,002 | n.a. | <0,01 | n.a. |
| Nickel | 58,70 | <0,05 | n.a. | <0,05 | n.a. | <0,05 | n.a. | <0,05 | n.a. | 0,058 | 0,00 |
| Chrom | 52,00 | <0,05 | n.a. | <0,05 | n.a. | <0,05 | n.a. | <0,05 | n.a. | 0,016 | 0,00 |
| **Summe basenbildend** | | | 85,55 | | 79,22 | | 92,86 | | 93,17 | | 137,41 |
| **Verhältnis** (Σ basenbildend) zu (Σ säurebildend) | | | 2,93 Ostara 00 | | 4,16 Desiree 00 | | 4,31 Planta 00 | | 4,89 Desiree 00 | | 4,10 Bintje 00 |

Tabelle 2a

12

| | mg / mmol | O1 Ostara Ernte 2001 | | D1 Desiree Ernte 2001 | | A1 Ackersegen 2001 | |
|---|---|---|---|---|---|---|---|
| | | mg / l | mmol / l | mg / l | mmol / l | mg / l | mmol / l |
| Sulfat | 96,06 | 728 | 7,58 | 405 | 4,22 | 274 | 2,85 |
| Phosphat | 94,97 | 520 | 5,48 | 597 | 6,29 | 454 | 4,78 |
| Chlorid | 35,45 | 514 | 14,50 | 175 | 4,94 | 95 | 2,68 |
| Nitrat | 62,01 | 137 | 2,21 | 91 | 1,47 | 14 | 0,23 |
| Malat | 132,10 | 1240 | 9,39 | 940 | 7,12 | 1400 | 10,60 |
| Lactat | 89,08 | 75 | 0,84 | 78 | 0,88 | 96 | 1,08 |
| Tartrat | 148,10 | <1 | n.a. | <1 | n.a. | <1 | n.a. |
| Citrat | 189,10 | 3300 | 17,45 | 3600 | 19,04 | 4000 | 21,15 |
| Isocitrat | 189,10 | 240 | 1,27 | 272 | 1,44 | 341 | 1,80 |
| L-Ascorbat | 175,05 | 2,4 | 0,01 | 0,8 | 0,00 | 1,9 | 0,01 |
| **Summe säurebildend** | | | **58,73** | | **45,38** | | **45,18** |
| Kalium | 39,10 | 2500 | 63,94 | 2390 | 61,13 | 2320 | 59,34 |
| Magnesium | 24,31 | 256 | 10,53 | 188 | 7,73 | 223 | 9,17 |
| Calcium | 40,08 | 35 | 0,87 | 45 | 1,12 | 53 | 1,32 |
| Natrium | 22,99 | 2,5 | 0,11 | 3,4 | 0,15 | 3,3 | 0,14 |
| Ammonium | 18,04 | <0,05 | n.a. | <0,05 | n.a. | <0,05 | n.a. |
| Eisen | 55,84 | 0,49 | 0,01 | 0,51 | 0,01 | 1,38 | 0,02 |
| Zink | 65,30 | 3,1 | 0,05 | 2,8 | 0,04 | 2,3 | 0,04 |
| Mangan | 54,94 | 1,35 | 0,02 | 1,42 | 0,03 | 1,44 | 0,03 |
| Kupfer | 63,54 | 1,3 | 0,02 | 1,1 | 0,02 | 1,3 | 0,02 |
| Selen | 78,90 | <0,002 | n.a. | <0,002 | n.a. | <0,002 | n.a. |
| Nickel | 58,70 | 0,08 | 0,00 | 0,1 | 0,00 | 0,09 | 0,00 |
| Chrom | 52,00 | <0,05 | n.a. | <0,05 | n.a. | <0,05 | n.a. |
| **Summe basenbildend** | | | **75,55** | | **70,23** | | **70,08** |
| **Verhältnis (Σ basenbildend) zu (Σ säurebildend)** | | | **1,29** Ostara 01 | | **1,55** Desiree 01 | | **1,55** Ackersegen 01 |

Tabelle 2b

| | mg/mmol | L1 Ostara Ernte 2000 mg/l | L1 mmol/l | O1 Ostara Ernte 2001 mg/l | O1 mmol/l | L2 Desiree Ernte 2000 mg/l | L2 mmol/l | D1 Desiree Ernte 2001 mg/l | D1 mmol/l |
|---|---|---|---|---|---|---|---|---|---|
| Sulfat | 96,06 | 1190 | 12,39 | 728 | 7,58 | 702 | 7,31 | 405 | 4,22 |
| Phosphat | 94,97 | 791 | 8,33 | 520 | 5,48 | 796 | 8,38 | 597 | 6,29 |
| Chlorid | 35,45 | 244 | 6,88 | 514 | 14,50 | 94 | 2,65 | 175 | 4,94 |
| Nitrat | 62,01 | 100 | 1,61 | 137 | 2,21 | 43 | 0,69 | 91 | 1,47 |
| Malat | 132,10 | | | 1240 | 9,39 | | | 940 | 7,12 |
| Lactat | 89,08 | | | 75 | 0,84 | | | 78 | 0,88 |
| Tartrat | 148,10 | | | <1 | n.a. | | | <1 | n.a. |
| Citrat | 189,10 | | | 3300 | 17,45 | | | 3600 | 19,04 |
| Isocitrat | 189,10 | | | 240 | 1,27 | | | 272 | 1,44 |
| L-Ascorbat | 175,05 | | | 2,4 | 0,01 | | | 0,8 | 0,00 |
| Σ säurebildend anorganisch | | | 29,21 | | 29,76 | | 19,03 | | 16,91 |
| Σ säurebildend gesamt | | | | | 58,73 | | | | 45,38 |
| Kalium | 39,10 | 2880 | 73,66 | 2500 | 63,94 | 2640 | 67,52 | 2390 | 61,13 |
| Magnesium | 24,31 | 257 | 10,57 | 256 | 10,53 | 252 | 10,37 | 188 | 7,73 |
| Calcium | 40,08 | 27,6 | 0,69 | 35 | 0,87 | 23,5 | 0,59 | 45 | 1,12 |
| Natrium | 22,99 | 11,8 | 0,51 | 2,5 | 0,11 | 13,4 | 0,58 | 3,4 | 0,15 |
| Ammonium | 18,04 | <0,05 | n.a. | <0,05 | n.a. | <0,05 | n.a. | <0,05 | n.a. |
| Eisen | 55,84 | 2,07 | 0,04 | 0,49 | 0,01 | 3,95 | 0,07 | 0,51 | 0,01 |
| Zink | 65,30 | 2,82 | 0,04 | 3,1 | 0,05 | 2,88 | 0,04 | 2,8 | 0,04 |
| Mangan | 54,94 | 1,35 | 0,02 | 1,35 | 0,02 | 1,82 | 0,03 | 1,42 | 0,03 |
| Kupfer | 63,54 | 0,85 | 0,01 | 1,3 | 0,02 | 1,4 | 0,02 | 1,1 | 0,02 |
| Selen | 78,90 | <0,002 | n.a. | <0,002 | n.a. | <0,002 | n.a. | <0,002 | n.a. |
| Nickel | 58,70 | <0,05 | n.a. | 0,08 | 0,00 | <0,05 | n.a. | 0,1 | 0,00 |
| Chrom | 52,00 | <0,05 | n.a. | <0,05 | n.a. | <0,05 | n.a. | <0,05 | n.a. |
| Σ basenbildend | | | 85,55 | | 75,55 | | 79,22 | | 70,23 |
| Verhältnis (Σ basenbildend) zu (Σ säurebildend anorganisch) | | | 2,93 Ostara 00 | | 2,54 Ostara 01 | | 4,16 Desiree 00 | | 4,15 Desiree 01 |

Tabelle 2c

Die Ergebnisse zeigen deutlich, dass zwar durch einen Wasserverlust während der Lagerung die absolute Konzentration der Ionen zunimmt, aber das für das zu entwickelnde Produkt wertbestimmende Verhältnis der Summe der Säure-bildenden Ionen zu den Basen-bildenden nicht signifikant beeinflusst wird.

Die Sortenwahl für die weiterführenden Versuche fiel im Hinblick auf das vorhandene Kartoffelmaterial zugunsten der Sorte Desiree aus. Hinsichtlich des wertbestimmenden Quotienten erwiesen sich die Sorten Desiree und Ackersegen zwar als gleichwertig, aber aufgrund anbau- und erntetechnischer Faktoren wurde der Sorte Desiree der Vorzug gegeben.

14

### 3. GEWINNUNG DER ELEKTROLYTE

Prinzipiell waren zwei alternative Gewinnungsmethoden zur Isolierung der basischen Elektrolyte in möglichst reiner Form gemäß dem Stand der Technik in Diskussion.

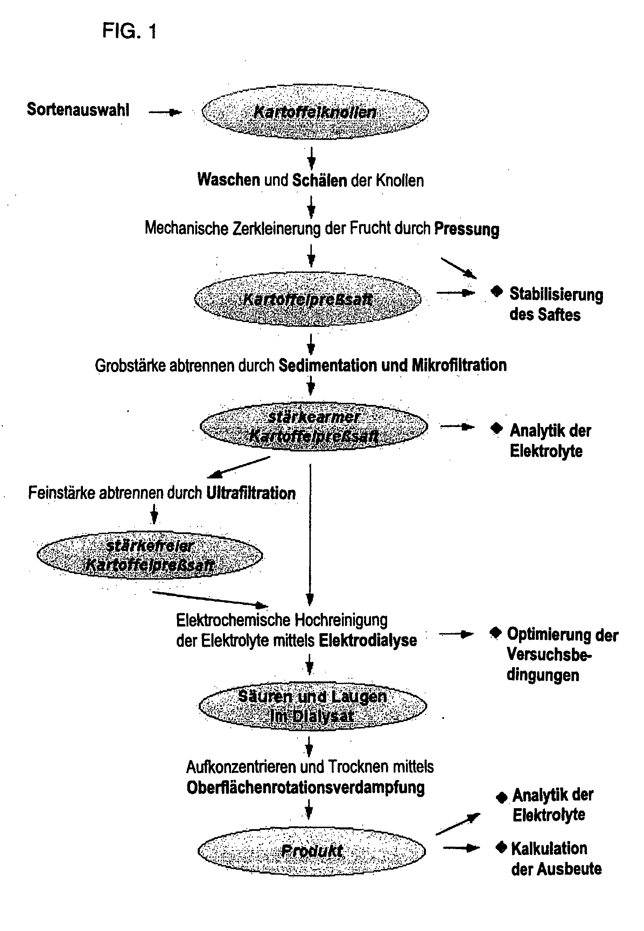

Einerseits wurde eine alleinige Abtrennung der nicht erwünschten Komponenten über den Trennparameter der Größe unter Anwendung diverser Filtrationsmethoden (Mikro-, Ultra- und Nanofiltration) erwogen. Da jedoch die äußerst komplexe Matrix eines Kartoffelpresssaftes sehr viele - auch niedermolekulare - Bestandteile aufweist, wie beispielsweise Kohlenhydrate in unterschiedlichen Polymerisationsgraden (Monomere, Dimere, Oligomere), freie Aminosäuren, niedermolekulare Peptide und Fettsäuren, stellte sich letztendlich eine Kombination zweier unterschiedlicher Trennverfahren als vorteilhaft dar: ein Filtrationsverfahren (Ultrafiltration) unter Ausnutzung des Trennparameters der Größe, gefolgt von einer Elektrodialyse, die primär die Ladung der zu isolierenden Elektrolyte ausnutzt, aber über die Auswahl der Membranen auch eine gewisse Selektion über das Verhältnis der Ladung bezogen auf die Größe zulässt (vgl. Fig. 1).

### 3.1 Material

Aufgrund der Ergebnisse der Analysen zur Sortenauswahl wurde die Sorte Desiree für die Pilotversuche ausgewählt:

Lungauer Speisekartoffeln
Erzeuger Nr.22
Sorte Desiree
Abpackdatum 28.9.01
Kartoffellager A-5580 Tamsweg

### 3.2 Methoden

### 3.2.1 Bereitung des Kartoffelpresssaftes

Die gewaschenen und geschälten Kartoffeln wurden mechanisch zerkleinert und in einem handelsüblichen Entsafter entsaftet. Die Hauptmenge der Kartoffelstärke wurde durch eine mehrstündige Sedimentation bei $+4\,^{o}C$ abgetrennt. Der so erhaltene Stärke-arme Kartoffelpresssaft wurde in einem Kartuschen-Filter mit einer Porengröße von 1 μm (Mikrofiltration) filtriert, um etwaige Faser- und Stärkerückstände zu entfernen und für die Ultrafiltrations- und/oder Elektrodialyseversuche eingesetzt.

### 3.2.2 Ultrafiltration

Da die ersten Versuche der Elektrodialyse - wie im folgenden Kapitel detailliert beschrieben - Probleme durch Belegung der Membranen, sogenannte "Fouling-Phänomene", zeigen können, wurde gemäß einem verbesserten Verfahren der Elektrodialyse eine Ultrafiltration des Stärke-armen Kartoffelpresssaftes vorgelagert, um höher molekulare Bestandteile noch effektiver abzutrennen.

Für einen Vorversuch, der zeigen sollte, ob wertbestimmende Ionen durch diesen Verfahrensschritt verloren gehen, wurde eine Rührzelle der Fa. Amicon mit einer Polysulfon-Membran einer Ausschlussgrenze von 1000 Da unter 2 bar Arbeitsdruck eingesetzt. Da die Ergebnisse deutlich zeigten, dass keinerlei wertbestimmende Elektrolyte verloren gehen, wurde ein Pilotversuch mit einem Plattenmodul, ebenfalls bestückt mit den oben genannten Polysulfon-Membranen mit einer Ausschlussgrenze von 1000 Da und 2 bar Arbeitsdruck unter periodischer Rückspülung eingesetzt.

### 3.2.3 Elektrodialyse

Technische Membranen, die in den bekannten Verfahren der Mikro- und Ultrafiltration eingesetzt werden, trennen gelöste Stoffe ausschließlich nach ihrer Teilchengröße, unabhängig von ihren chemischen und elektrochemischen Eigenschaften.

Im Gegensatz dazu ist die Elektrodialyse ein Membranverfahren unter Einwirkung des elektrischen Feldes. Dieses Feld wirkt auf alle ionogenen Komponenten und kann zur Stofftrennung eingesetzt werden.

Als Trennelement dienen auch hier Membranen, die aber nicht den Trennparameter Größe zur Selektion verwenden, sondern die Ladung der Moleküle. Die verschiedenen Ionen werden durch selektive Ionenaustauschmembranen in Kationen und Anionen separiert, wobei als treibende Kraft ein elektrisches Potentialgefälle wirkt. Infolge des angelegten elektrischen Potentials wandern die gelösten Ionen durch die Membranen auf die entsprechende Elektrode zu, so dass auf der einen Seite der Membrane eine verdünnte Lösung, das sogenannte Diluat zurückbleibt und auf der anderen Seite die Ionen angereichert werden, im sogenannten Konzentrat.

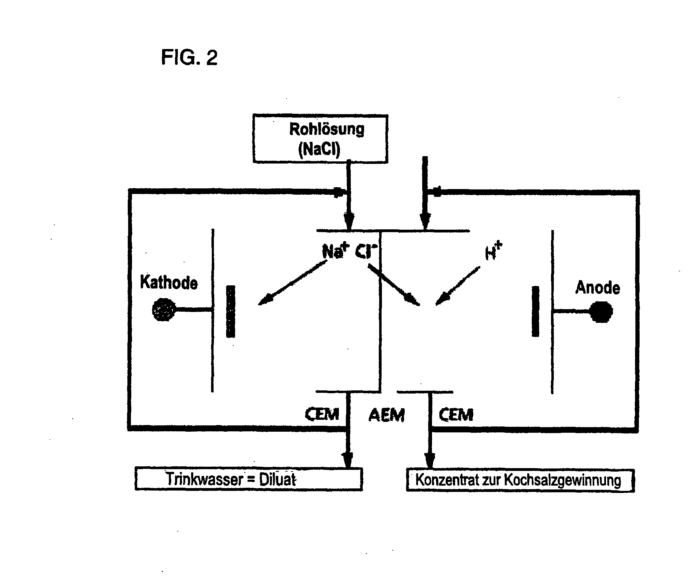

Das Funktionsprinzip dieses Verfahrens ist in Fig. 2 schematisch kurz anhand der Elektrodialyse einer Kochsalzlösung dargestellt.

Die technische Realisierung dieses Verfahrens verwendet Membranstapel, wobei die einzelnen Membranen mittels Abstandhalter und die so entstandenen Räume mit Hilfe von Dichtrahmen voneinander und von den Elektrodenkammern getrennt sind. Durch einen alternierenden Einsatz von Kationen- und Anionen-Austausch-Membranen resultiert eine Anreicherung der Ionen im sogenannten Kozentrat-Kreislauf und eine entsprechende Abnahme an Ladungsträgern im sogenannten Diluat-Kreislauf.

Die Pilotversuche wurden unter Verwendung einer Membranelektrolysezelle Stapeltyp 36 mit 36 cm$^2$ eff. Membranfläche/Membran, 10 Zellpaare, insgesamt 0,0756 m$^2$ eff. Membranfläche durchgeführt.

### 3.3 Ergebnisse und Diskussion

In der ersten Phase der Pilotversuche (Versuch 1 und 2) sollte getestet werden, welche Membranen für unsere Fragestellung günstige Trenneigenschaften aufweisen. Primär wurden 2 verschiedene Membrantypen untersucht: einerseits die normalen Standardmembranen und andererseits ein Membrantyp, der sowohl eine unspezifische Diffusion von nicht geladenen Partikeln als auch die Wanderung höhermolekularer Elektrolyte (wie beispielsweise organische Anionen, freie Aminosäuren,...) erschwert, sogenannte "Low Diffusion-Membrane".

### 3.3.1 Pilotversuch 1

| | | | |
|---|---|---|---|
| Probenmaterial | Kartoffelpresssaft der Sorte Desiree aufgearbeitet wie unter 3.2.1 beschrieben: ◆ Sedimentation ◆ Mikrofiltration mit 1μm Porengröße | | |
| Elektrolysezelle | ◆ Stapeltyp 36 ◆ 36 cm$^2$ eff. Membranfläche / Membran | | |
| Membrane | ◆ Standardmembrane ◆ 10 Zellpaare ◆ insgesamt 0,0756 m$^2$ eff. Membranfläche | | |
| Lösungen zu Beginn des Versuches | | Volumen | Flussrate |
| Diluat-seitig | Kartoffelpresssaft | 3 l | 70 l/h |
| Konzentrat-seitig | Stadtwasser | 3 l | 70 l/h |
| Elektroden-Spülung | Na$_2$SO$_4$ 8,3 mS/cm | 2 l | 300 l/h |
| Versuchsbedingungen | ◆ konstante Betriebsspannung von 21 V ◆ Umpolung für 30 Sekunden alle 30 Minuten | | |
| Probennahme | ◆ alle 30 Minuten | | |

Zur Dokumentation der Ionenwanderung während des Versuches wurden einerseits repräsentative Messdaten, wie die Stromstärke (I, [A]), die Leitfähigkeiten sowohl im Diluat als auch im Konzentrat (Lf, [mS/cm]), als auch der pH-Wert erfasst. Zur Veranschaulichung sind die Leitfähigkeiten des Diluates und des Konzentrates über den Zeitraum des gesamten Versuches in der folgenden Abbildung graphisch erfasst.

Andererseits wurden zeitabhängige Proben (6 Proben in Intervallen von je 30 Minuten) gezogen und bei diesem Vorversuch auf den als Summenparameter für organische Bestandteile zu interpretierenden DOC (gemessen als NPOC) wie bereits zuvor beschrieben untersucht (Ergebnisse: Tabelle 3).

Tabelle 3

| | Stadt-Wasser, | Versuch 1 30 Min | Versuch 1 60 Min | Versuch 1 90 Min | Versuch 1 120 Min | Versuch 1 150 Min | Versuch 1 180 Min | Proben-Material |
|---|---|---|---|---|---|---|---|---|
| | mg/l | mg/l | mg/l | mg/l | mg/l | mg/l | mg/l | mg/l |
| DOC | 1,1 | 245 | 576 | 792 | 850 | 1530 | 1450 | 9490 |

Auffällig bei diesem Membrantyp (Standardmembrane) war, dass die Anionen-Austauscher-Membranen massiv verschmutzt und unter Rillenbildung gequollen waren. Der Belag ließ sich nur teilweise durch die normalen Reinigungsvorgänge abspülen.

**3.3.2 Pilotversuch 2**

| Probenmaterial | Kartoffelpresssaft der Sorte Desiree aufgearbeitet wie unter 3.2.1 beschrieben:<br><br>♦ Sedimentation und<br><br>♦ Mikrofiltration mit 1μm Porengröße |
|---|---|
| Elektrolysezelle | ♦ Stapeltyp 36<br><br>♦ 36 $cm^2$ eff. Membranfläche / Membran |
| Membrane | ♦ "Low Diffusion-Membrane"<br><br>♦ 10 Zellpaare<br><br>♦ insgesamt 0,0756 $m^2$ eff. Membranfläche |

| Lösungen zu Beginn des Versuches | | Volumen | Flussrate |
|---|---|---|---|
| Diluat-seitig | Kartoffelpresssaft | 3 l | 70 l/h |
| Konzentrat-seitig | Stadtwasser | 3 l | 70 l/h |
| Elektroden-Spülung | $Na_2SO_4$ 8,3 mS/cm | 2 l | 300 l/h |

| Versuchsbedingungen | ♦ konstante Betriebsspannung von 21 V<br><br>♦ Umpolung für 30 Sekunden alle 30 Minuten |
|---|---|
| Probennahme | ♦ alle 30 Minuten |

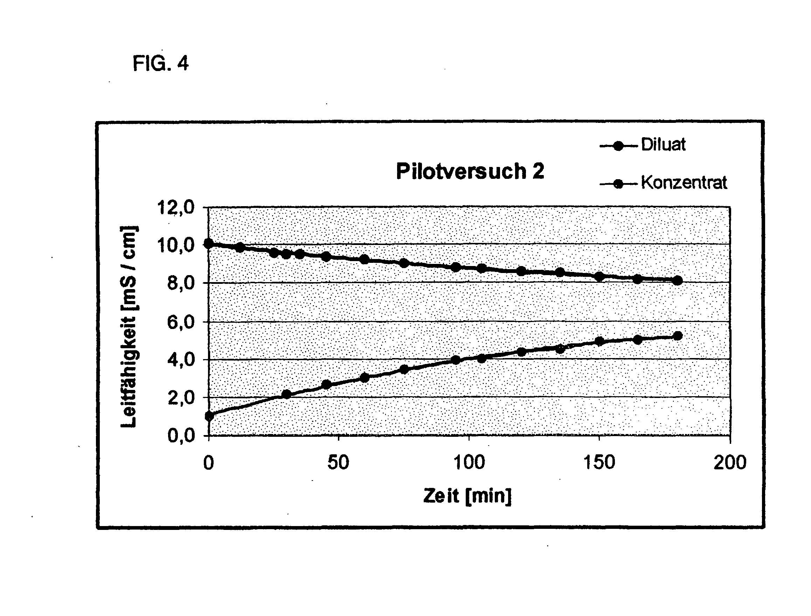

Auch bei diesem Versuch wurde die Stromstärke, die Leitfähigkeiten sowohl im Diluat als auch im Konzentrat und der pH-Wert erfasst. Die Abbildung der zeitabhängigen Veränderungen der Leitfähigkeiten des Diluates und des Konzentrates in Fig. 4 zeigt bereits ein markant anderes Profil in der Ionenwanderung im Vergleich zum ersten Versuch.

Auch bei diesem Versuch wurden zeitabhängige Proben im Intervall von 30 Minuten gezogen und auf ihre Zusammensetzung untersucht. Da bereits im Verlauf des Versuches die eindeutig bessere Eignung der "Low-Diffusion-Membrane" erkennbar war, wurde bei diesen Proben eine umfassende Analytik sowohl der organischen Bestandteile als auch der anorganischen Kat- und Anionen durchgeführt (vgl. Tabelle 4).

| | mg/mmol | Stadt-Wasser mg/l | Versuch 2 30 Min mg/l | mmol/l | Versuch 2 60 Min mg/l | mmol/l | Versuch 2 90 Min mg/l | mmol/l | Versuch 2 120 Min mg/l | mmol/l | Versuch 2 150 Min mg/l | mmol/l | Versuch 2 180 Min mg/l | mmol/l | Proben-Material mg/l | mmol/l |
|---|---|---|---|---|---|---|---|---|---|---|---|---|---|---|---|---|
| Sulfat | 96,06 | 33 | 71 | 0,74 | 95 | 0,99 | 110 | 1,15 | 114 | 1,19 | 142 | 1,48 | 153 | 1,59 | 215 | 2,24 |
| Phosphat | 94,97 | 0,03 | 108 | 1,14 | 175 | 1,84 | 239 | 2,52 | 255 | 2,69 | 342 | 3,60 | 376 | 3,96 | 601 | 6,33 |
| Chlorid | 35,45 | 5,4 | 299 | 8,43 | 364 | 10,27 | 383 | 10,80 | 360 | 10,16 | 403 | 11,37 | 398 | 11,23 | 270 | 7,62 |
| Nitrat | 62,01 | 4,3 | <1,0 | n.a. | <1,0 | n.a. | <1,0 | n.a. | <1,0 | n.a. | <1,0 | n.a. | <1,0 | n.a. | <1,0 | n.a. |
| Malat | 132,10 | | n.a. | n.a. | n.a. | n.a. | n.a. | n.a. | n.a. | n.a. | n.a. | n.a. | n.a. | n.a. | n.a. | n.a. |
| Lactat | 89,08 | | n.a. | n.a. | n.a. | n.a. | n.a. | n.a. | n.a. | n.a. | n.a. | n.a. | n.a. | n.a. | n.a. | n.a. |
| Tartrat | 148,10 | | n.a. | n.a. | n.a. | n.a. | n.a. | n.a. | n.a. | n.a. | n.a. | n.a. | n.a. | n.a. | n.a. | n.a. |
| Citrat | 189,10 | | n.a. | n.a. | n.a. | n.a. | n.a. | n.a. | n.a. | n.a. | n.a. | n.a. | n.a. | n.a. | n.a. | n.a. |
| Isocitrat | 189,10 | | n.a. | n.a. | n.a. | n.a. | n.a. | n.a. | n.a. | n.a. | n.a. | n.a. | n.a. | n.a. | n.a. | n.a. |
| Ascorbat | 175,05 | | n.a. | n.a. | n.a. | n.a. | n.a. | n.a. | n.a. | n.a. | n.a. | n.a. | n.a. | n.a. | n.a. | n.a. |
| DOC | | 5,1 | 51,9 | | 83,1 | | 117,0 | | 141,0 | | 230,0 | | 351,0 | | 9490,0 | |
| Σ säurebildend | | | | 10,31 | | 13,10 | | 14,47 | | 14,03 | | 16,45 | | 16,78 | | 16,18 |
| Kalium | 39,10 | 1,3 | 522 | 13,35 | 750 | 19,18 | 942 | 24,09 | 1030 | 26,34 | 1060 | 27,11 | 1140 | 29,16 | 3110 | 79,54 |
| Magnesium | 24,31 | 8,1 | 12 | 0,49 | 13 | 0,53 | 14 | 0,58 | 14 | 0,58 | 16 | 0,66 | 17 | 0,70 | 215 | 8,84 |
| Calcium | 40,08 | 47,7 | 27 | 0,67 | 54 | 1,35 | 42 | 1,05 | 35 | 0,87 | 26 | 0,65 | <10 | n.a. | 20 | 0,50 |
| Natrium | 22,99 | 4,4 | 69 | 3,00 | 75 | 3,26 | 95 | 4,13 | 103 | 4,48 | 120 | 5,22 | 125 | 5,44 | 2,8 | 0,12 |
| Ammonium | 18,04 | <0,01 | n.a. | n.a. | n.a. | n.a. | n.a. | n.a. | n.a. | n.a. | n.a. | n.a. | n.a. | n.a. | n.a. | n.a. |
| Eisen | 55,84 | <0,005 | 0,64 | 0,01 | 0,70 | 0,01 | 0,43 | 0,01 | 0,47 | 0,01 | 0,88 | 0,02 | 0,39 | 0,01 | 4,69 | 0,08 |
| Zink | 65,30 | 0,0005 | 0,15 | 0,00 | 0,08 | 0,00 | 0,09 | 0,00 | 0,06 | 0,00 | 0,06 | 0,00 | <0,05 | n.a. | 2,6 | 0,04 |
| Mangan | 54,94 | 0,0004 | 1,0 | 0,02 | <0,05 | n.a. | <0,05 | n.a. | <0,05 | n.a. | 0,05 | 0,00 | 0,05 | 0,00 | 1,9 | 0,03 |
| Kupfer | 63,54 | 0,001 | 0,24 | 0,00 | 0,35 | 0,01 | 0,32 | 0,01 | 0,29 | 0,00 | 0,30 | 0,00 | 0,19 | 0,00 | 1,20 | 0,02 |
| Selen | 78,90 | 0,0014 | <0,002 | n.a. | <0,002 | n.a. | <0,002 | n.a. | <0,002 | n.a. | <0,002 | n.a. | <0,002 | n.a. | <0,002 | n.a. |
| Nickel | 58,70 | <0,001 | <0,05 | n.a. | <0,05 | n.a. | 0,09 | 0,00 | 0,06 | 0,00 | 0,06 | 0,00 | <0,05 | n.a. | 2,6 | 0,04 |
| Chrom | 52,00 | <0,0005 | <0,05 | n.a. | <0,05 | n.a. | <0,05 | n.a. | <0,05 | n.a. | <0,05 | n.a. | <0,05 | n.a. | <0,05 | n.a. |
| Σ basenbildend | | | | 17,55 | | 24,35 | | 29,86 | | 32,29 | | 33,66 | | 35,30 | | 89,23 |
| Quotient (Σ basenbildend)/(Σ säurebildend) | | | | 1,70 30 Min | | 1,86 60 Min | | 2,06 90 Min | | 2,30 120 Min | | 2,05 150 Min | | 2,10 180 Min | | 5,51 Proben Material |

Tabelle 4

Der im ersten Versuch beobachtete "Fouling-Prozess", also die Belegung der Anionen-Austauscher-Membranen konnte beim Einsatz der "Low Diffusion-Membranen" nur in weit geringerem Ausmaß beobachtet werden.

Weiters ließ sich diese Belegung auch einfach mit den herkömmlichen Reinigungsmethoden entfernen und der Formverändernde Prozess der Quellung konnte überhaupt nicht festgestellt werden.

**3.3.3. Zusammenfassend haben die Ergebnisse der ersten beiden Versuche Folgendes gezeigt:**

- Die im zweiten Versuch eingesetzten "Low-Diffusion-Membranen" zeigen für die gegenständliche Fragestellung ein besseres Trennverhalten, da sie den Transport von unerwünschten organischen Anteilen in das Konzentrat signifikant erschweren. Der DOC als Summenparameter für diese Anteile ist bei der Verwendung der Standardmembranen nach 180 Minuten Versuchsdauer bei 1450 mg/l, mit ‚Low Diffusion-Membranen nach der selben Versuchsdauer unter ansonsten identischen Bedingungen hingegen bei 351 mg/l. Jener des eingesetzten Kartoffelpresssaftes zum Vergleich lag bei 9490 mg/l.
- Auch in Bezug auf das unerwünschte "Fouling-Verhalten" ist eindeutig den "Low Diffusion-Membranen" der Vorzug zu geben. Einerseits sind hier keinerlei Formveränderungen durch Quellprozesse zu beobachten und andererseits tritt eine Belegung der Membranen in weit geringerem Ausmaß auf.
- Betrachtet man nun die detaillierte Analyse der ionogenen Substanzen im Konzentrat des zweiten Versuches, so kristallisieren sich folgende Fakten heraus:

- Die Wanderung der anorganischen Anionen entspricht weitgehend dem laut theoretischem Hintergrund erwarteten Muster, dass einwertige Ionen schneller wandern als zweiwertige und letztere wieder schneller als dreiwertige. Im Vergleich zum eingesetzten Kartoffelpresssaft ist nach einer Versuchsdauer von 180 Minuten Phosphat zu 62% und Sulfat zu 70% gewandert. Die detektierten Konzentrationen an Chlorid dagegen liegen weit über den erwarteten und sind sogar höher als jene im ursprünglichen Kartoffelpresssaft, nämlich 3.6 mmol/l im Überschuss. Wie im folgenden Kapitel betreffend der Kationen noch dargelegt, ist ebenso ein signifikanter Überschuss an Natrium (5.3 mmol/l) im Konzentrat nachzuweisen. Diese bei erster Betrachtung widersprüchlich erscheinenden Fakten sind jedoch durch einen Ionenaustausch an den eingesetzten Membranen zu erklären. Die Ionenaustausch-Kapazität der Membranen beträgt laut Herstellerangaben 1,5 mmol/g trockener Membranen. Unter der Annahme, dass 1 Membran etwa 1 g Trockengewicht hat und je 10 Kationen- und Anionen-Membranen im Versuchsaufbau verwendet wurden, ist ein potentieller Eintrag der an der Membran gebundenen Gegenionen Natrium und Chlorid im Ausmaß von je 15 mmol möglich und unter Berücksichtigung des Gesamtvolumens an Konzentrat von 3 l auch in der Größenordnung des detektierten Überschusses. Ein derartiges Phänomen der Loslösung der Hersteller-seitig an die Ionenaustauscher-Oberflächen gebundenen Ionen ist denkbar unter der durchaus plausiblen Annahme, dass die Bindungsstellen im Laufe des Versuches durch etwaige höher-molekulare Anteile belegt wurden, die auch rein optisch als Belag erkennbar waren.
- Betrachtet man das Wanderverhalten der Kationen, so fällt - abgesehen vom bereits diskutierten Überschuss an Natrium - auf, dass sämtliche Kationen signifikant in ihrer Diffusion durch die Membran gehindert werden. Beispielsweise Kalium, das ja aufgrund seiner Einwertigkeit relativ vollständig wandern sollte, ist nur zu 37% im Konzentrat. Bei den höherwertigen Kationen - zwar primär bei den Wert bestimmenden Ionen Calcium und Magnesium, aber auch ganz deutlich bei den Spurenelementen Eisen, Kupfer, Mangan, Nickel und Zink - sind die Verhältnisse noch drastischer. Das detektierbare Calcium und Magnesium stammt zur Gänze aus dem zu Versuchsbeginn eingesetzten Stadtwasser und spiegelt offensichtlich das Faktum wider, dass die einwertigen Kationen schlecht und die mehrwertigen gar nicht gewandert sind.
- Phänomene dieser Art sind in der Elektrodialyse bekannt und in speziellen Fällen sogar erwünscht. Beispielsweise werden speziell beschichtete Membranen als "selektive Ionenaustausch-Membranen" eingesetzt, um selektiv einwertige Ionen von mehrwertigen zu trennen. Da in unserem Versuch offensichtlich die Trenneigenschaft der Membranen in ähnlicher Weise durch Belegung mit Begleitstoffen aus dem Kartoffelpresssaft verändert wurde, wurde für den nächsten Versuch ein weiterer Filtrationsschritt zur Vorreinigung des Saftes, nämlich eine Ultrafiltration mit einer Ausschlussgrenze von 1000 Da pilotiert.

**3.3.4 Pilotversuch 3**

Wie bereits dargelegt, haben die ersten beiden Pilotversuche gezeigt, dass der bis dato mittels Sedimentation und Mikrofiltration mit einer Porengröße von 1 μm vorgereinigte Kartoffelpresssaft noch weiter aufbereitet werden muss, um das unerwünschte und die Trenneigenschaften verändernde Phänomen des "Foulings" möglichst zu unterbinden.

Deshalb wurde das Probenmaterial für den nächsten Elektrodialyse-Pilotversuch mit einer Ultrafiltration mit einer Ausschlussgrenze von 1000 Da vorfiltriert.

Ein Vorversuch zu diesem Filtrationsschritt wurde - wie bereits vorher beschrieben - in einer Rührzelle durchgeführt, um festzustellen, ob Wert-bestimmende Ionen durch diesen Verfahrensschritt verloren gehen. Die potentielle Möglichkeit eines Verlustes vor allem der zweiwertigen Kationen Magnesium und Calcium durch Komplexbildung mit Molekülen mit visceralen OH-Gruppen, wie sie in Kohlenhydraten vorkommen, sollte verifiziert werden.

Da die Ergebnisse jedoch deutlich zeigen, dass keinerlei Wert-bestimmende Elektrolyte verloren gehen (wie in der Ergebnistabelle des 3. Pilotversuches (Tabelle 5) zu erkennen ist), wurde die für einen weiteren Elektrodialyse-Schritt erforderliche Menge in einem Plattenmodul, ebenfalls bestückt mit den oben genannten Polysulfon-Membranen mit einer Ausschlussgrenze von 1000 Da filtriert.

Auch bei diesem Membranverfahren wurden die bereits bei der Elektrodialyse beobachteten "Fouling-Phänomene" festgestellt. Periodische Rückspülung während der Filtration war erforderlich.

Der 3. **Pilotversuch** der Elektrodialyse wurde schließlich mit dem Filtrat der Ultrafiltration unter den in den ersten beiden Versuchen optimierten Rahmenbedingungen (Einsatz von "Low-Diffusion-Membranen", periodische Umpolung) wie folgt durchgeführt:

| | |
|---|---|
| Probenmaterial | Kartoffelpresssaft der Sorte Desiree aufgearbeitet wie unter 3.2.1 und 3.2.2 beschrieben: <br><br> ♦ Sedimentation <br><br> ♦ Mikrofiltration mit 1 µm Porengröße <br><br> ♦ Ultrafiltration mit einer Ausschlussgrenze von 1000 Da |
| Elektrolysezelle | ♦ Stapeltyp 36 <br><br> ♦ 36 cm$^2$ eff. Membranfläche / Membran |
| Membrane | ♦ "Low Diffusion-Membrane" <br><br> ♦ 10 Zellpaare <br><br> ♦ insgesamt 0,0756 m$^2$ eff. Membranfläche |

| Lösungen zu Beginn des Versuches | | Volumen | Flussrate |
|---|---|---|---|
| Diluat-seitig | Kartoffelpresssaft | 2 l | 75 l/h |
| Konzentrat-seitig | VE-Wasser | 2 l | 75 l/h |
| Elektroden-Spülung | Na$_2$SO$_4$ 8,3 mS/cm | 2 l | 200 l/h |

| | |
|---|---|
| Versuchsbedingungen | ♦ konstante Betriebsspannung von 21 V <br><br> ♦ Umpolung für 30 Sekunden alle 30 Minuten |
| Versuchsdauer | ♦ 180 Minuten |

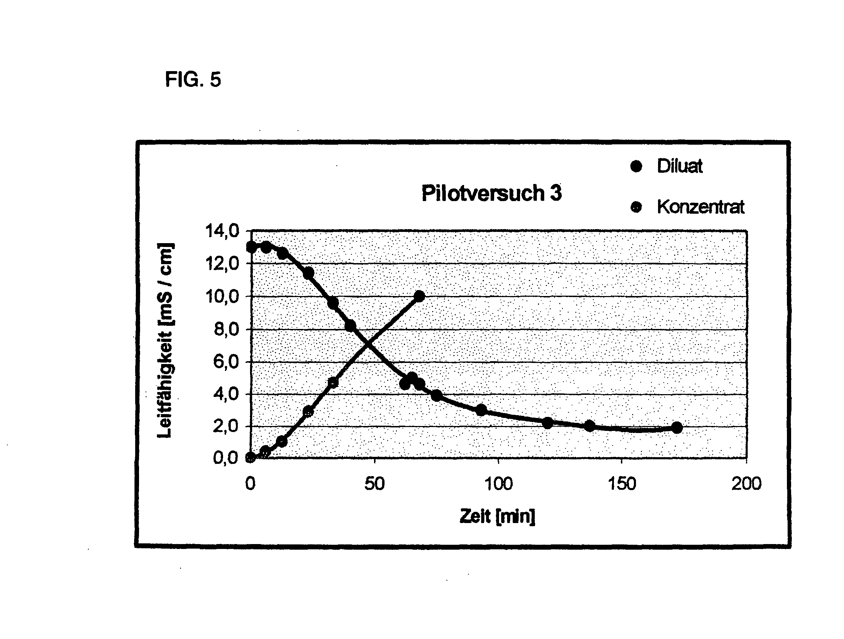

Wie bei den vorigen Versuchen wurden auch hier die Stromstärke und die Leitfähigkeiten sowohl im Diluat als auch im Konzentrat erfasst und letztere graphisch dargestellt (Fig. 5).

Auffallend bei diesem Versuch ist der sigmoide Kurvenverlauf im Vergleich zu den beiden anderen Versuchen, die eher einen hyperbolischen Verlauf ergaben. Dieses Phänomen ist durch das auf der Konzentrat-Seite zu Beginn des Versuches eingesetzte Wasser unterschiedlicher Qualität und Leitfähigkeit zu erklären. Bei den Versuchen 1 und 2 wurde gegen Stadtwasser dialysiert, das die laut Analyse bekannte Ionenzusammensetzung enthält und aufgrund derer bereits zum Zeitpunkt 0 Leitfähigkeit vorhanden ist. Im Gegensatz dazu wurde der letzte Versuch mit VE-Wasser (vollentsalztem Wasser) gestartet, welches zu Versuchsbeginn keinerlei Leitfähigkeit aufweist und zu dem graphisch zu beobachtenden verzögerten Beginn in der Elektrodialyse führt.

Tabelle 5

| | mg / mmol | Versuch 3 180 Min Diluat | | Versuch 3 180 Min Konzentrat | | % (Konzentrat/Diluat) | Proben-Material ultrafiltriert | | Versuch 2 180 Min Konzentrat | |
|---|---|---|---|---|---|---|---|---|---|---|
| | | mg/l | mmol/l | mg/l | mmol/l | | mg/l | mmol/l | mg/l | mmol/l |
| Sulfat | 96,06 | 66 | 0,69 | 189 | 1,97 | 74,1 | 241 | 2,51 | 153 | 1,59 |
| Phosphat | 94,97 | 141 | 1,48 | 520 | 5,48 | 78,7 | 625 | 6,58 | 376 | 3,96 |
| Chlorid | 35,45 | 36 | 1,02 | 513 | 14,47 | 93,4 | 298 | 8,41 | 398 | 11,23 |
| Nitrat | 62,01 | 1,7 | 0,03 | 3 | 0,05 | 63,8 | <1,0 | n.a. | <1,0 | n.a. |
| Malat | 132,10 | 69 | 0,52 | 737 | 5,58 | 91,4 | n.a. | n.a. | n.a. | n.a. |
| Lactat | 89,08 | <1 | n.a. | <1 | n.a. | n.a. | n.a. | n.a. | n.a. | n.a. |
| Tartrat | 148,10 | <1 | n.a. | 36 | 0,24 | n.a. | n.a. | n.a. | n.a. | n.a. |
| Citrat | 189,10 | 281 | 1,49 | 452 | 2,39 | 61,7 | n.a. | n.a. | n.a. | n.a. |
| Isocitrat | 189,10 | 67 | 0,35 | 166 | 0,88 | 71,2 | n.a. | n.a. | n.a. | n.a. |
| Ascorbat | 175,05 | 0,4 | 0,00 | 0,3 | 0,00 | 42,9 | n.a. | n.a. | n.a. | n.a. |
| DOC | | 4760 | | 4000 | | 45,7 | n.a. | | 351,0 | |
| Σ säurebildend anorganisch | | | 3,21 | | 21,96 | | | 17,50 | | 16,78 |
| Σ säurebildend gesamt | | | 5,58 | | 31,05 | | | | | |
| Kalium | 39,10 | 516 | 13,20 | 3500 | 89,51 | 87,2 | 3890 | 99,49 | 1140 | 29,16 |
| Magnesium | 24,31 | 36 | 1,48 | 210 | 8,64 | 85,4 | 272 | 11,19 | 17 | 0,70 |
| Calcium | 40,08 | <10 | n.a. | 72 | 1,80 | 100,0 | 124 | 3,09 | <10 | n.a. |
| Natrium | 22,99 | 37 | 1,61 | 189 | 8,22 | 83,6 | 15,6 | 0,68 | 125 | 5,44 |
| Ammonium | 18,04 | n.a. | n.a. | n.a. | n.a. | n.a. | n.a. | n.a. | n.a. | n.a. |
| Eisen | 55,84 | 24 | 0,43 | 57 | 1,02 | 70,4 | 0,44 | 0,01 | 0,39 | 0,01 |
| Zink | 65,30 | 0,98 | 0,02 | 2,36 | 0,04 | 70,7 | 2,5 | 0,04 | <0,05 | n.a. |
| Mangan | 54,94 | 0,33 | 0,01 | 1,69 | 0,03 | 83,7 | 1,29 | 0,02 | 0,05 | 0,00 |
| Kupfer | 63,54 | 0,65 | 0,01 | 0,2 | 0,00 | 23,5 | 0,63 | 0,01 | 0,19 | 0,00 |
| Selen | 78,90 | <0,002 | n.a. | <0,002 | n.a. | n.a. | <0,002 | n.a. | <0,002 | n.a. |
| Nickel | 58,70 | 1,03 | 0,02 | 0,26 | 0,00 | 20,2 | 0,06 | 0,00 | <0,05 | n.a. |
| Chrom | 52,00 | 0,72 | 0,01 | 0,24 | 0,00 | 25,0 | <0,05 | n.a. | <0,05 | n.a. |
| Σ basenbildend | | | 16,78 | | 109,27 | | | 114,53 | | 35,30 |
| Verhältnis (Σ basenbildend) zu (Σ säurebildend anorganisch) (Σ säurebildend gesamt) | | | 5,22 / 3,01 | | 4,98 / 3,52 | | | 6,55 | | 2,10 |

Das Konzentrat dieses Pilotversuches wurde einerseits hinsichtlich der Ionenzusammensetzung analysiert und andererseits mittels Oberflächenrotationsverdampfung eingeengt. Unter den Versuchsbedingungen der Laboranlage (40°C und ein Vakuum bis 30 mbar) wurden aus 360 ml Konzentrat 9,96 g amorphes Produkt gewonnen.

Hier sei noch anzumerken, dass für eine großtechnische Umsetzung natürlich das gegenständliche, im Labormaßstab gewählte Verfahren zur Trocknung nicht adäquat ist. In diesem Fall ist selbstverständlich ein Verfahren, wie beispielsweise die Sprüh- oder Walzentrocknung zu pilotieren und optimieren.

Betrachtet man nun die Analysenergebnisse des Pilotversuches 3, der eine weitere Vorreinigung des Kartoffelpresssaftes mittels Ultrafiltration vor der Elektrodialyse inkludiert hat, so werden folgende Fakten deutlich:

- Die im zweiten Versuch aufgetretenen Probleme durch Belegung der Ionenaustauschmembranen konnten durch die Vorreinigung des Saftes mittels Ultrafiltration mit einer Ausschlussgrenze von 1000 Da weitgehend gelöst werden. Offensichtlich wurden durch diesen Verfahrensschritt jene höhermolekularen Anteile aus der Probenmatrix effizient entfernt, die bei den ersten beiden Versuchen zu den problematischen "Fouling-Phänomenen" und den dadurch bedingten Änderungen der Trenneigenschaften der Membranen geführt haben, die sich ja ganz massiv auf die Kationenwanderung ausgewirkt haben.
- Ferner ist im Gegensatz zum zweiten Pilotversuch durch die Ultrafiltration des Saftes auch das Problem des Ionenaustausches und der dadurch bedingten Loslösung der Gegenionen Chlorid und Natrium aus den Membranen durch Matrixbestandteile der Probe nicht mehr zu beobachten. Vielmehr entspricht die Wanderung der anorganischen Anionen bei diesem Versuch den Erwartungen. Sulfat beispielsweise ist zu 74%, Phosphat zu 79% und Chlorid zu 93% gewandert. Auch die organischen Anionen wandern entsprechend ihrer Ladung und Größe; Malat, das kleinste der untersuchten Anionen zu 91%, Citrat und Isocitrat zu 62 und 72% und Ascorbat zu 42%.
- Die verbesserte Wanderung der Ionen hat sich natürlich auch auf die im Summenparameter des DOC erfassten organischen Bestandteile der Probenmatrix ausgewirkt. So liegt der DOC diesmal bei 4000 mg/l im Konzentrat und ist damit deutlich höher als im zweiten Pilotversuch. Hier sei jedoch angemerkt, dass in diesem Wert auch die detailliert quantifizierten organischen Anionen mit erfasst sind. Zu den qualitativ und quantitativ nicht erfassten Komponenten, die ebenfalls im Konzentrat zu finden sind und ihren Beitrag zu diesem Summenparameter leisten, gehören vermutlich primär Aspartat und Glutamat.
- Betrachtet man das Wanderverhalten der Kationen, so ist eindeutig erkennbar, dass der zusätzliche Verfahrensschritt der Ultrafiltration die Probleme durch das "Fouling-Verhalten" im zweiten Versuch gelöst hat. Die für das zu entwickelnde Produkt wertbestimmenden Kationen wandern durch die letztendlich angewandte Kombination an Verfahrensschritten äußerst zufriedenstellend. Sogar die zweiwertigen Kationen Magnesium und Calcium wandern zu 85% beziehungsweise sogar vollständig. Kalium wandert zu 87%, Natrium zu 84% und die mengenmäßig weniger dominierenden Ionen, wie Eisen, Zink und Mangan wandern zwischen 70 und 84%.
- Diese äußerst erfreuliche Verbesserung der Leistung der Elektrodialyse spiegelt sich auch in den bereits eingangs definierten Quotienten aus der Summe der Basen-bildenden zu den Säure-bildenden Elektrolyten wider. Vergleicht man beispielsweise den Quotienten zwischen dem 2. und 3. Versuch, so ist durch diese Innovation eine Steigerung von 2,10 auf 4,98, also eine *Steigerung um 237%* zu verzeichnen! Hier sei anzumerken, das die oben genannten Quotienten auf die anorganischen Anionen bezogen sind, da bei Versuch 2 die organischen Anionen nicht detailliert analysiert wurden.
- Vergleicht man den Quotienten, der auch die analysierten organischen Anionen berücksichtigt, so zeigt unser Endprodukt einen Wert von 3,52 im Vergleich zum nicht verarbeiteten Kartoffelsaft, der Im Falle der eingesetzten Sorte Desiree einen Wert von 1,55 aufweist. Hier ist also eine Zunahme von 227% zu verzeichnen!

**Patentansprüche**

1. Verfahren zur lebensmitteltechnischen Gewinnung von Kartoffelsaft-Produkten, **gekennzeichnet durch** die folgenden Schritte:

    - Bereitstellen von Kartoffelpresssaft,
    - Abfiltrieren von Faser- oder Stärkerückständen über Mikrofilter,
    - vorzugsweise Ultrafiltration des Filtrates
    - Elektrodialyse des Mikro- bzw. Ultrafiltrates und
    - gegebenenfalls Trocknen des Elektrodialysates unter Zugabe von silikathältigen Trägerstoffen.

2. Verfahren nach Anspruch 1, **dadurch gekennzeichnet, dass** dem Kartoffelpresssaft Stabilisierungsmittel, vorzugsweise natürliche Antioxidantien, insbesondere Zitronensaft oder Zitronensaft-Produkte, zugegeben werden.

3. Verfahren nach Anspruch 1 oder 2, **dadurch gekennzeichnet, dass** der Kartoffelpresssaft aus Kartoffeln bereitgestellt wird, die ein Verhältnis von Basen-bildenden zu Säure-bildenden Komponenten von zumindest 1,5, bevorzugter von über 3,5, aufweisen, insbesondere Kartoffelpresssaft aus den Sorten Desiree und Ackersegen.

**4.** Verfahren nach einem der Ansprüche 1 bis 3, **dadurch gekennzeichnet, dass** die Ultrafiltration mit einem Ultrafilter mit einer Ausschlussgrenze von unter 100.000 Da, bevorzugt von unter 10.000 Da, insbesondere von etwa 1.000 Da, durchgeführt wird.

**5.** Verfahren nach einem der Ansprüche 1 bis 4, **dadurch gekennzeichnet, dass** die Elektrodialyse unter Verwendung eines Membranstapels, insbesondere eines Membranstapels von Niedrig-diffusionsmembranen, durchgeführt wird.

**6.** Verfahren nach einem der Ansprüche 1 bis 5, **dadurch gekennzeichnet, dass** das Trocknen unter Zugabe von hochdispersem Siliziumdioxid durchgeführt wird.

**7.** Verfahren nach einem der Ansprüche 1 bis 6, **dadurch gekenn zeichnet, dass** dem erhaltenen und gegebenenfalls getrockneten Kartoffelsaft-Produkt weitere Mittel zugesetzt werden, vorzugsweise zumindest ein weiterer Gemüse- oder Fruchtsaft, ein oder mehrere Stabilisierungsmittel, insbesondere natürliche Antioxidantien, ein oder mehrere, insbesondere natürliche, Geschmacks- oder Farbstoffe, ein oder mehrere Verdickungs-, Rekonstituierungs- oder Elektrolytmittel oder Kombinationen dieser Mittel sowie Vitamine, Mineralstoffe, Spurenelemente, sekundäre Pflanzenstoffe oder Kombinationen dieser Mittel.

**8.** Verfahren nach einem der Ansprüche 1 bis 7, **dadurch gekennzeichnet, dass** das Trocknen durch Sprüh- oder Walztrocken erfolgt.

**9.** Kartoffelsaft-Produkt, erhältlich nach einem der Ansprüche 1 bis 8.

**10.** Kartoffelsaft-Produkt nach Anspruch 9, **dadurch gekennzeichnet, dass** es organische Bestandteile von 1000 mg/l oder mehr, vorzugsweise 2000 mg/l oder mehr, insbesondere 4000 mg/l oder mehr, bestimmt als nicht ausblasbaren organischen Kohlenstoff, enthält.

**11.** Kartoffelsaft-Produkt nach Anspruch 9 oder 10, **dadurch gekennzeichnet, dass** es ein Verhältnis von Basenbildenden zu Säure-bildenden Komponenten von zumindest 2,5, bevorzugter von über 4, insbesondere von über 6, aufweist.

**12.** Verwendung eines Kartoffelsaft-Produktes nach einem der Ansprüche 9 bis 11 zur Herstellung eines Mittels zur Regulierung des Säure/Basen-Haushaltes.

**Claims**

**1.** A method for the food-technological preparation of potato juice products, **characterised by** the following steps:

- provision of a pressed potato juice,
- separation of fibre or starch residues by filtration through microfilters,
- preferably ultrafiltration of the filtrate,
- electrodialysis of the microfiltrate or ultrafiltrate, respectively, and
- optionally drying of the electrodialysate under the addition of silicate-containing carrier substances.

**2.** A method according to claim 1, **characterised in that** stabilizers, preferably natural antioxidants, in particular lemon juice or lemon juice products, are added to the pressed potato juice.

**3.** A method according to claim 1 or 2, **characterised in that** the pressed potato juice is prepared from potatoes having a ratio of base-forming to acid-forming components of at least 1.5, more preferred above 3.5, in particular pressed potato juice from the varieties Desiree and Ackersegen.

**4.** A method according to any one of claims 1 to 3, **characterised in that** said ultrafiltration is carried out using an ultrafilter having a cut-off of below 100,000 Da, preferably below 10,000 Da, in particular approximately 1,000 Da.

**5.** A method according to any one of claims 1 to 4, **characterised in that** said electrodialysis is carried out using a membrane stack, in particular a membrane stack of low diffusion membranes.

6. A method according to any one of claims 1 to 5, **characterised in that** said drying is carried out under the addition of highly disperse silicon dioxide.

7. A method according to any one of claims 1 to 6, **character ised in that** the potato juice product obtained and optionally dried is supplemented with additional agents, preferably at least one additional vegetable or fruit juice, one or several stabilisers, in particular natural antioxidants, one or several particularly natural flavouring or colouring agents, one or several thickening, reconstitution or electrolytic agents or combinations of said agents as well as vitamins, mineral substances, trace elements, secondary plant substances or combinations of said agents.

8. A method according to any one of claims 1 to 7, **characterised in that** said drying is effected by spray-drying or drum-drying.

9. A potato juice product obtainable according to any one of claims 1 to 8.

10. A potato juice product according to claim 9, **characterised in that** it comprises 1000 mg/l or more, preferably 2000 mg/l or more, in particular 4000 mg/l or more, of organic components, determined as non-purgeable organic carbon.

11. A potato juice product according to claim 9 or 10, **characterised in that** it has a ratio of base-forming to acid-forming components of at least 2.5, more preferred above 4, in particular above 6.

12. The use of a potato juice product according to any one of claims 9 to 11 for the preparation of an agent intended to regulate the acid/base balance.


**Revendications**

1. Procédé d'obtention de produits à base de jus de pomme de terre par des techniques relatives aux produits alimentaires, **caractérisé par** les étapes suivantes :

   - mise à disposition de jus de pomme de terre obtenu par pression,
   - filtration des résidus de fibre et d'amidon par des microfiltres,
   - de préférence, ultrafiltration du filtrat,
   - électrodialyse du filtrat de microfiltration ou d'ultrafiltration et
   - le cas échéant, séchage du produit d'électrodialyse moyennant addition de substances supports contenant des silicates.

2. Procédé selon la revendication 1, **caractérisé en ce que** l'on ajoute au jus de pommes de terre obtenu par pression, des agents de stabilisation, de préférence des antioxydants naturels, en particulier du jus de citron ou des produits à base de jus de citron.

3. Procédé selon la revendication 1 ou 2, **caractérisé en ce que** le jus de pommes de terre obtenu par pression est mis à disposition à partir de pommes de terre qui présentent un rapport des composants générateurs de bases aux composants générateurs d'acides d'au moins 1,5, de préférence de plus de 3,5, en particulier du jus de pommes de terre obtenu par pression des variétés Desiree et Ackersegen.

4. Procédé selon l'une des revendications 1 à 3, **caractérisé en ce que** l'ultrafiltration est exécutée avec un ultrafiltre avec une limite d'exclusion de moins de 100000 Da, de préférence de moins de 10000 Da, en particulier d'environ 1000 Da.

5. Procédé selon l'une des revendications 1 à 4, **caractérisé en ce que** l'électrodialyse est exécutée moyennant l'utilisation d'une pile de membranes, en particulier d'une pile de membranes à basse diffusion.

6. Procédé selon l'une des revendications 1 à 5, **caractérisé en ce que** le séchage est exécuté moyennant l'addition de dioxyde de silicium hautement dispersé.

7. Procédé selon l'une des revendications 1 à 6, **caractérisé en ce que** l'on ajoute au produit de jus de pommes de terre obtenu et séché le cas échéant d'autres agents, de préférence au moins un autre jus de légume ou de fruit, un ou plusieurs agents stabilisateurs, en particulier des antioxydants naturels, un ou plusieurs aromates ou colorants, en particulier naturels, un ou plusieurs agents épaississants, reconstituants ou électrolytes ou des combinaisons de ces agents, ainsi que des vitamines, des substances minérales, des oligo-éléments, des substances secondaires de plantes ou des combinaisons de ces agents.

8. Procédé selon l'une des revendications 1 à 7, **caractérisé en ce que** le séchage s'effectue par un séchage par pulvérisation ou sur cylindres.

9. Produit de jus de pommes de terre pouvant être obtenu selon l'une des revendications 1 à 8.

10. Produit de jus de pommes de terre selon la revendication 9, **caractérisé en ce qu'**il contient des composés organiques de 1 000 mg/l ou plus, de préférence 2 000 mg/l ou plus, en particulier de 4 000 mg/l ou plus, déterminés comme carbone organique ne pouvant être éliminé par soufflage.

11. Produit de jus de pommes de terre selon la revendication 9 ou 10, **caractérisé en ce qu'**il présente un rapport des composants générateurs de bases aux composants générateurs d'acides d'au moins 2,5, plus préférablement de plus de 4, en particulier de plus de 6.

12. Utilisation d'un produit de jus de pommes de terre selon l'une des revendications 9 à 11 par la préparation d'un produit pour régulariser l'équilibre acido-basique.

## FIG. 1

Sortenauswahl →  **Kartoffelknollen**

↓

**Waschen** und **Schälen** der Knollen

↓

Mechanische Zerkleinerung der Frucht durch **Pressung**

↓

**Kartoffelpreßsaft** → ◆ **Stabilisierung des Saftes**

↓

Grobstärke abtrennen durch **Sedimentation und Mikrofiltration**

↓

**stärkearmer Kartoffelpreßsaft** → ◆ **Analytik der Elektrolyte**

Feinstärke abtrennen durch **Ultrafiltration**

↓

**stärkefreier Kartoffelpreßsaft**

↓

Elektrochemische Hochreinigung
der Elektrolyte mittels **Elektrodialyse** → ◆ **Optimierung der Versuchsbedingungen**

↓

**Säuren und Laugen im Dialysat**

↓

Aufkonzentrieren und Trocknen mittels
**Oberflächenrotationsverdampfung**

↓

**Produkt** → ◆ **Analytik der Elektrolyte**

→ ◆ **Kalkulation der Ausbeute**

FIG. 2

Rohlösung (NaCl)

Kathode

Anode

Na⁺ Cl⁻

H⁺

CEM    AEM    CEM

Trinkwasser = Diluat

Konzentrat zur Kochsalzgewinnung

EP 1 494 546 B1

28

FIG. 3

Pilotversuch 1

Diluat
Konzentrat

Leitfähigkeit [mS/cm]
12,0  10,0  8,0  6,0  4,0  2,0  0,0

Zeit [min]
0   50   100   150   200

FIG. 4

Pilotversuch 2

Legend:
—●— Diluat
—●— Konzentrat

Y-axis: Leitfähigkeit [mS / cm]
X-axis: Zeit [min]

EP 1 494 546 B1

FIG. 5